# EUROPEAN PATENT APPLICATION

(11) **EP 4 786 590 A1**
(43) Date of publication of application: **05.08.2026**
(21) Application number: 24873051.7
(22) Date of filing: 27.09.2024
(51) Int. Cl.: C12N 15/11, C12N 15/67

(54) **NUCLEIC ACID MOLECULE COMPRISING MODIFIED POLYADENYLATION SEQUENCE**

(30) Priority: 27.09.2023 KR 20230130729
(71) Applicant: Hanmi Pharm. Co., Ltd., Hwaseong-si, Gyeonggi-do 18536 (KR)
(72) Inventor: KIM, Jeong Woo, Hwaseong-si, Gyeonggi-do 18469 (KR); SHIN, Seung Hyun, Hwaseong-si, Gyeonggi-do 18469 (KR); LEE, Jong Soo, Hwaseong-si, Gyeonggi-do 18469 (KR); LIM, Chang Gyu, Hwaseong-si, Gyeonggi-do 18469 (KR); JANG, Doo Seo, Hwaseong-si, Gyeonggi-do 18469 (KR); HAN, Young Jin, Hwaseong-si, Gyeonggi-do 18469 (KR); HEO, Yong Ho, Hwaseong-si, Gyeonggi-do 18469 (KR)
(74) Representative: Santarelli
(86) International application number: PCT/KR2024/014754
(87) International publication number: WO 2025/071344

(57) **Abstract**

Provided are an mRNA molecule including a modified polyadenylation sequence, a composition for delivering the mRNA or a product expressed therefrom to a subject, a method for delivering the mRNA or a product expressed therefrom to a host cell, a DNA molecule encoding the mRNA molecule, and uses thereof.

## Description

### Technical Field

The present disclosure relates to a nucleic acid molecule including a modified polyadenylation (poly(A)) sequence, a nucleic acid molecule encoding the same, and uses thereof.

### Background Art

RNA, such as *in vitro* transcribed RNA (IVT-RNA), has been proposed as an alternative to the therapeutic use of DNA. However, the injection of RNA for clinical applications may be significantly limited by the short half-life of RNA.

In addition, IVT vectors may be used as templates for *in vitro* transcription. Such an IVT vector may include a 5' RNA polymerase promoter, a gene of interest flanked by a 5' UTR, a 3' UTR, or both a 5' UTR and a 3' UTR, and a 3' polyadenylataion cassette including A nucleotides.

The 3' poly(A) of RNA is important for the nuclear export, RNA stability, and translation efficiency of eukaryotic mRNA. It is known that the 3' poly(A) sequence shortens over time, and when it becomes sufficiently short, the RNA is degraded by enzymes.

Therefore, despite the aforementioned prior art, there is a need for a nucleic acid molecule including a modified polyadenylation sequence, a nucleic acid molecule encoding the same, and uses thereof.

### Disclosure

### Technical Problem

An aspect provides an mRNA molecule including a modified polyadenylation sequence.

Another aspect provides a DNA encoding the mRNA.

Another aspect provides a method of propagating the DNA molecule.

Another aspect provides a method of obtaining an RNA.

Another aspect provides a method of obtaining a polypeptide.

Another aspect provides a composition or a kit for delivering an mRNA or a product expressed therefrom to a subject, including the mRNA molecule.

Another aspect provides a method of delivering an mRNA or a product expressed therefrom to a host cell, including introducing the mRNA into the host cell.

### Technical Solution

A first aspect provides an mRNA molecule including a modified polyadenylation sequence, wherein the modified polyadenylation sequence includes: (a) a first sequence including one or more microRNA binding sequences; (b) a second sequence of 20 or fewer consecutive A nucleotides, located 5' of the first sequence; and (c) a third sequence of 80 or more consecutive A nucleotides, located 3'of the first sequence.

As used herein, the terms "polyadenylation sequence," "poly(A)," or "poly(A) tail" refer to a region of mRNA located downstream of, for example, directly downstream (i.e., 3' to) the 3' UTR, which includes a plurality of consecutive adenosine monophosphates. The poly(A) tail may include 10 to 1000 adenosine monophosphates. For example, the poly(A) tail may include 10, 20, 30, 40, 50, 60, 70, 80, 90, 100, 110, 120, 130, 140, 150, 160, 170, 190, 200, 210, 220, 230, 240, 250, 260, 270, 280, 290, 300, 350, 400, 450, 500, 600, 700, 800, 900, or 1,000 or more A nucleotides. The poly(A) tail may include, for example, 50 to 1000, 60 to 800, 80 to 600, 100 to 500, 50 to 500, 60 to 500, 70 to 500, 80 to 500, 90 to 500, 100 to 500, 50 to 400, 60 to 400, 70 to 400, 80 to 400, 90 to 400, 100 to 400, 50 to 300, 60 to 300, 70 to 300, 80 to 300, 90 to 300, 100 to 300, 50 to 200, 50 to 250, 60 to 200, 70 to It may comprise 200, 80 to 200, 90 to 200, 100 to 200, 50 to 100, 60 to 100, 70 to 100, 80 to 100, 90 to 100, or 150 to 250 consecutive A nucleotides. In vivo, such as in a cell or subject, the poly(A) tail may serve to protect mRNA from attack by enzymes, for example, those in the cytoplasm, and to aid in transcription termination, nuclear export of mRNA, and translation.

Polyadenylation refers to the addition of a poly(A) sequence to primary transcript RNA. The poly(A) sequence is composed of a plurality of adenosine monophosphates. That is, a poly(A) sequence is a stretch of RNA containing only adenine bases. The poly(A) sequence is important for nuclear export, translation, and the stability of mRNA. The poly(A) sequence shortens over time, and when it becomes sufficiently short, the mRNA may be degraded by enzymes.

In the mRNA molecule, the modified polyadenylation sequence may include one or more disruptions in a consecutive A sequence, to which one or more microRNA binding sequences are linked. The modified polyadenylation sequence may perform the inherent functions of a polyadenylation sequence with equal or superior efficiency compared to an unmodified polyadenylation sequence. The original functions may include aiding in, for example, enhancing, the nuclear export, translation, and stability of mRNA.

As used herein, the term "microRNA" or "miRNA" is used in the sense known in the art. The microRNA may be a long non-coding RNA of 19-25 nucleotides in length that binds to an mRNA molecule to downregulate gene expression. The microRNA may bind to the 3' UTR of the mRNA. The microRNA may include a sequence complementary to a region of a target mRNA to which it binds. The complementary sequence may be a perfectly or near-perfectly complementary sequence. The downregulation of gene expression may include downregulation by decreasing the stability of a nucleic acid molecule or by inhibiting translation.

The term "microRNA binding sequence" may be any nucleotide sequence to which a microRNA binds or associates. The term "binding" may follow traditional Watson-Crick hybridization rules or may reflect any stable association between the microRNA and a target sequence at or adjacent to the microRNA binding sequence. The microRNA binding sequence may be a microRNA target sequence, a microRNA sequence, a microRNA seed sequence, or a combination thereof. The microRNA binding sequence may be a known sequence. A microRNA sequence includes a "seed sequence." The seed sequence may be a sequence in the region of positions 2-8 of a mature microRNA. The seed sequence may have complete Watson-Crick complementarity to the microRNA target sequence. An example of the microRNA sequence may include positions 2-8 or 2-7 of a mature microRNA. The microRNA sequence may include 7 nucleotides (e.g., nucleotides 2-8 of a mature microRNA), and the seed complementary site of the corresponding miRNA target may be flanked by an adenine (A) opposite to position 1 of the microRNA. Additionally, the microRNA sequence may include 6 nucleotides (e.g., nucleotides 2 to 7 of a mature microRNA), and the seed-complementary site of the corresponding miRNA target may be flanked by an adenine (A) opposite to position 1 of the microRNA. The microRNA binding sequence may be naturally occurring or artificially produced.

In the first sequence of the modified polyadenylation sequence, when two or more microRNA binding sequences are included, they may be identical to or different from each other. Furthermore, when two or more microRNA binding sequences are included, they may have microRNA target sequences, microRNA recognition sequences, or seed sequences thereof that are identical to or different from each other. For example, two or more microRNA binding sequences may be arranged in the form of ABAB, AABB, or ABBA. The two or more microRNA binding sequences may be directly linked to each other without a spacer. Additionally, the two or more microRNA binding sequences may be linked to each other via a spacer. The first sequence may be consisting of one or more microRNA binding sequences. The spacer may be a modified or unmodified nucleotide sequence.

In the nucleic acid molecule, the microRNA may be tumor-specific. The microRNA may be upregulated or downregulated in tumor cells compared to normal cells. The microRNA binding sequence may inhibit a tumor by binding to a tumor-specific miRNA, a seed sequence thereof, or a miRNA target sequence in vivo.

The microRNA may be selected from the group consisting of: miR-340, miR-133a, miR-34b, miR-19, miR-200, miR-15, miR-16, miR-1298, miR-663a, miR-449a, miR-138, miR-126, miR-10a, miR-10b, miR-140-3p, miR-140-5p, miR-204, miR-424, miR-34a, miR-193a-3p, miR-455-5p, miR-455-3p, miR-9, miR-10a, miR-148a, miR-488, miR-196a1, miR-182, miR-96, miR-193b, miR-27a, miR-196b, miR-10b, miR-29b2, miR-23a, miR-107, miR-542-3p, miR-93, miR-365a-4, miR-450a, miR-100, miR-105, miR-363, miR-105, miR-106b, miR-15b, miR-21, miR-376c, miR-93, miR-99b, miR-155, miR-33a, miR-876-3p, miR-362-3p, miR-25, let-7i, miR-423-3p, miR-16-2, miR-29a, miR-30d, miR-320, miR-181c, miR-128a, miR-21, let-7d, miR-450b-5p, miR-371-5p, miR-1245, miR-335, miR-492, miR-874, miR-30b, miR-193a-5p, miR-602, miR-346, miR-663, miR-25, miR-219-5p6, miR-184, miR-135a7, miR-584, miR-665, miR-638, miR-503, miR-628-3p, miR-381, miR-78, miR-92b, miR-149, miR-135b, miR-302d, miR-498, miR-766, miR-1389, miR-623, miR-519c-5p, miR-182, miR-494, miR-129-5p10, miR-513-5p, miR-200b, miR-634, miR-654-5p, miR-518b, miR-658, miR-373, miR-30c-2, miR-130a, miR-557, miR-551a, miR-637, miR-518c, miR-525-5p, miR-596, miR-552, miR-625, miR-183, miR-187, miR-544, miR-891a, miR-519e, miR-933, miR-939, miR-214, miR-671-5p, miR-137, miR-92b, miR-525-3p, miR-19a, and miR-409-5p. An example of the microRNA may be one that is downregulated in tumor cells. The downregulated miR may be miR-34a. However, differential expression of miRs in cancer cells may vary depending on the cancer type.

In the second sequence of the modified polyadenylation sequence, the length of the consecutive A nucleotides may be any length that provides sufficient stability and/or translation to the mRNA obtained upon transcription, given the microRNA binding sequences, the number thereof, and the length of the consecutive A nucleotides of the third sequence. For example, the length of the consecutive A nucleotides of the second sequence may be 1 to 20, 2 to 20, 3 to 20, 4 to 20, 5 to 20, 6 to 20, 7 to 20, 8 to 20, 9 to 20, 10 to 20, 11 to 20, 12 to 20, 13 to 20, 14 to 20, 15 to 20, 16 to 20, 17 to 20, 18 to 20, 19 to 20, 20, 1 to 19, 2 to 19, 3 to 19, 4 to 19, 5 to 19, 6 to 19, 7 to 19, 8 to 19, 9 to 19, 10 to 19, 11 to 19, 12 to 19, 13 to 19, 14 to 19, 15 to 19, 16 to 19, 17 to 19, 18 to 19, 19, 1 to 18, 2 to 18, 3 to 18, 4 to 18, 5 to 18, 6 to 18, 7 to 18, 8 to 18, 9 to 18, 10 to 18, 11 to 18, 12 to 18, 13 to 18, 14 to 18, 15 to 18, 16 to 18, 17 to 18, 18, 1 to 17, 2 to 17, 3 to 17, 4 to 17, 5 to 17, 6 to 17, 7 to 17, 8 to 17, 9 to 17, 10 to 17, 11 to 17, 12 to 17, 13 to 17, 14 to 17, 15 to 17, 16 to 17, 17, 1 to 16, 2 to 16, 3 to 16, 4 to 16, 5 to 16, 6 to 16, 7 to 16, 8 to 16, 9 to 16, 10 to 16, 11 to 16, 12 to 16, 13 to 16, 14 to 16, 15 to 16, 16, 1 to 15, 2 to 15, 3 to 15, 4 to 15, 5 to 15, 6 to 15, 7 to 15, 8 to 15, 9 to 15, 10 to 15, 11 to 15, 12 to 15, 13 to 15, 14 to 15, 15, 1 to 14, 2 to 14, 3 to 14, 4 to 14, 5 to 14, 6 to 14, 7 to 14, 8 to 14, 9 to 14, 10 to 14, 11 to 14, 12 to 14, 13 to 14, 14, 1 to 13, 2 to 13, 3 to 13, 4 to 13, 5 to 13, 6 to 13, 7 to 13, 8 to 13, 9 to 13, 10 to 13, 11 to 13, 12 to 13, 13, 1 to 12, 2 to 12, 3 to 12, 4 to 12, 5 to 12, 6 to 12, 7 to 12, 8 to 12, 9 to 12, 10 to 12, 11 to 12, 12, 1 to 11, 2 to 11, 3 to 11, 4 to 11, 5 to 11, 6 to 11, 7 to 11, 8 to 11, 9 to 11, 10 to 11, 11, 1 to 10, 2 to 10, 3 to 10, 4 to 10, 5 to 10, 6 to 10, 7 to 10, 8 to 10, 9 to 10, 10, 1 to 9, 2 to 9, 3 to 9, 4 to 9, 5 to 9, 6 to 9, 7 to 9, 8 to 9, 9, 1 to 8, 2 to 8, 3 to 8, 4 to 8, 5 to 8, 6 to 8, 7 to 8, 8, 1 to 7, 2 to 7, 3 to 7, 4 to 7, 5 to 7, 6 to 7, 7, 1 to 6, 2 to 6, 3 to 6, 4 to 6, 5 to 6, 6, 1 to 5, 2 to 5, 3 to 5, 4 to 5, 5, 1 to 4, 2 to 4, 3 to 4, 4, 1 to 3, 2 to 3, 3, 1 to 2, 2, or 1 nucleotide.

In the third sequence of the modified polyadenylation sequence, the length of the consecutive A nucleotides may be any length that provides sufficient stability and/or translation to the mRNA obtained upon transcription, given the microRNA binding sequences, the number thereof, and the length of the consecutive A nucleotides of the second sequence. For example, the length of the consecutive A nucleotides of the third sequence may be 80 to 150, 80 to 140, 80 to 130, 80 to 120, 80 to 110, 90 to 150, 90 to 140, 90 to 130, 90 to 120, or 90 to 110 nucleotides.

In the mRNA molecule, the length of the modified polyadenylation sequence may vary depending on the number of microRNA binding sequences. For example, the length of the modified polyadenylation sequence may be a length increased by a length corresponding to the number of microRNA binding sequences introduced, relative to the length of the polyadenylation sequence described in the section for "polyadenylation sequence." For example, when the length of the polyadenylation sequence is 10 to 1,000, 20 to 500, 30 to 500, 50 to 100, 80 to 160, 150 to 250, 100 to 300, 40 to 120, or 120 to 200 nucleotides, and when 1, 2, 3, 4, or 5 microRNA binding sequences are included, the modified polyadenylation sequence may have a sequence length of: 29 to 1,025, 39 to 525, 49 to 525, 69 to 125, 99 to 185, 169 to 275, 119 to 325, 59 to 145, 139 to 225, 48 to 1,050, 58 to 550, 68 to 550, 88 to 150, 118 to 210, 188 to 300, 138 to 350, 78 to 170, 158 to 250; 67 to 1,075, 77 to 575, 87 to 575, 107 to 175, 137 to 235, 207 to 325, 157 to 375, 97 to 195, 177 to 275, 86 to 1,100, 96 to 600, 106 to 600, 126 to 200, 156 to 260, 226 to 350, 176 to 400, 116 to 220, 196 to 300, 105 to 1,125, 115 to 625, 125 to 625, 145 to 225, 175 to 285, 245 to 375, 195 to 425, 135 to 245, or 215 to 325 nucleotides. The lengths of the modified polyadenylation sequence are described by way of example and are not limited to the lengths.

In the mRNA molecule, the one or more microRNA binding sequences may be located in a region of positions 21 to 39, 21 to 45, 21 to 70, 21 to 95, 21 to 120, 21 to 145, 11 to 39, 11 to 45, 11 to 70, 11 to 95, 11 to 120, or 11 to 145 of the modified polyadenylation sequence.

The mRNA molecule may include a sequence encoding a polypeptide or an expression regulatory sequence.

The sequence encoding a polypeptide, the expression regulatory sequence, and the modified polyadenylation sequence may be operably linked to each other.

As used herein, the term "open reading frame (ORF)" or "a sequence encoding a polypeptide" refers to a continuous region of a nucleic acid that encodes a polypeptide, beginning with a start codon, for example, a methionine codon (ATG), and ending with a stop codon, for example, TAA, TAG, or TGA.

As used herein, the term "operatively linked" (or "operably linked") refers to the linkage of nucleotide sequences on a single nucleic acid fragment such that the function of one is affected by the other. For example, a promoter is operably linked to a coding sequence (e.g., an ORF) if it is capable of affecting the expression of the coding sequence (i.e., if the coding sequence is under the transcriptional control of the promoter). A coding sequence may be operably linked to a regulatory sequence in a sense or antisense orientation. As used herein, unless otherwise stated, each sequence included in a nucleic acid molecule is understood to be operatively linked.

The expression regulatory sequence may be a translational regulatory sequence or a sequence involved in the stabilization of RNA. The expression regulatory sequence may be located upstream or downstream of the sequence encoding the polypeptide. The expression regulatory sequence may be located downstream of the sequence encoding the polypeptide and upstream of the modified polyadenylation sequence.

The expression regulatory sequence may be a Kozak sequence, a ribosome binding site, or a UTR. The UTR may be a 5' UTR, a 3' UTR, or both a 5' UTR and a 3' UTR. The 5' UTR, 3' UTR, or both the 5' UTR and 3' UTR may be homologous or heterologous to the sequence encoding the polypeptide. The 5' UTR may be a structured UTR. That is, the 5' UTR may have a three-dimensional structure, such as a stem-loop structure.

In the mRNA molecule, the 5' UTR may be a native human p53 5' UTR or a native human OX40L 5' UTR, the polypeptide may be a human p53 or human OX40L, and the 3' UTR may be a native human cytochrome P450 2E1 3' UTR.

The 5'UTR or 3'UTR may be one found in mRNA expressed in any tissue cell. The tissue may be a tissue where cancer is present. The cancer may be a blood cancer or a solid cancer. The tissue may be liver, muscle, brain, breast, colon, lung, pancreas, ovary, skin, or blood. The tissue cell may be, for example, a liver cell, an endothelial cell, a muscle cell, a blood cell, or an adipose tissue cell. The 5' UTR may be derived from an mRNA encoding a protein selected from the group consisting of p53, OX40L, albumin, serum amyloid A, apolipoprotein A/B/E, transferrin, alpha-fetoprotein, erythropoietin, factor VIII, MyoD, myosin, myoglobin, myogenin, herculin, Tie-1, CD36, C/EBP, AML1, G-CSF, GM-CSF, CD11b, MSR, Fr-1, i-NOS, CD45, CD18, CD36, GLUT4, ACRP30, adiponectin, P-A/B/C/D, ORM1, HPX, FGA, CYP2E1, C3, ASC, APOA2, ALB, AGXT, α-globin, β-globin, tyrosine hydroxylase, and collagen. The 5' UTR may be derived from, for example, an mRNA encoding human p53, human OX40L, or human C3 protein. In addition, the 3' UTR may be derived from an mRNA encoding human CYP450 2E1, human OX40L, or human C3 protein. The 3' UTR may be located 5' to a polyadenylation sequence.

The expression regulatory sequence may be a sequence homologous or heterologous to the sequence encoding the polypeptide.

The polypeptide may be any polypeptide having anti-cancer activity. The polypeptide having anti-cancer activity may have anti-cancer activity by being involved in the cell cycle regulation of the cancer cell itself, or may have anti-cancer activity through an activity of stimulating immune cells. The immune cells may include B cells, NK cells, T cells, or mast cells. The polypeptide having anti-cancer activity may be non-antigenic in a host cell or a subject including the same. The polypeptide having anti-cancer activity may be an antibody, a hormone, a cytokine, an enzyme, a derivative thereof, or a combination thereof. Additionally, the polypeptide having anti-cancer activity may inhibit the proliferation of cancer cells. The polypeptide may be a recombinant one including a fusion protein. The sequence encoding the polypeptide may be codon-optimized. The codon optimization may vary depending on the host cell into which the nucleic acid molecule is introduced. Additionally, the sequence encoding the polypeptide may include a degenerate sequence.

The polypeptide may exhibit a pharmacological effect by forming a trimer. The polypeptide may be fused with an element that promotes the formation of a trimer formed after translation. The element may be a polypeptide that promotes trimer formation. The element may be Mannan-binding lectin (MBL) or a fragment thereof, or ferritin or a fragment thereof. The fragment may be the alpha-helix region of MBL (position in the protein primary sequence: "110-129"). The fragment may be a heavy chain region of ferritin (position in the protein primary sequence: "15-161"). The polypeptide may be fused with one or more elements. The polypeptide may be fused with one or more elements via a linker. The linker may be a peptide linker. The peptide linker may be a linker comprising G4S. The peptide linker may be a linker including G4S, (G4S)2, (G4S)3, (G4S)4, (G4S)5, or (G4S)6. The polypeptide may include a structure of polypeptide-linker-MBL-linker-ferritin or MBL-linker-polypeptide.

In the mRNA molecule, the sequence encoding the polypeptide and the modified polyadenylation sequence may be transcribed under the control of a promoter to be generated as a common transcript. The modified polyadenylation sequence may be located at the 3' end of the common transcript.

The mRNA molecule may include one or more modified nucleotides. The modified nucleotide may be a pseudouridine analogue. The pseudouridine may be 1-methylpseudouridine. The modified nucleotide may further include 5-methylcytidine. In the mRNA molecule, all U may be substituted with 1-methylpseudouridine.

In the mRNA molecule, the sequence encoding the polypeptide may be codon-optimized.

Additionally, the mRNA may include one or more 5'-cap structures. As used herein, the term "cap" refers to a cap structure found at the 5'-end of an mRNA molecule. The cap is typically composed of a guanosine nucleotide linked to the mRNA via a 5'-to-5' triphosphate linkage. This guanosine is methylated at the 7-position. The term "conventional 5'-cap" refers to a naturally occurring RNA 5'-cap, such as 7-methylguanosine (m7G). As used herein, the term "5'-cap" includes modified 5'-cap analogs that are similar to an RNA cap structure and, when attached, possess the ability to stabilize RNA, for example, in vivo and/or in a cell. The one or more 5'-cap structures may be selected from the group consisting of 7-methylguanosine (m7G), ARCA, inosine, N1-methyl-guanosine, 2'-fluoro-guanosine, 7-deaza-guanosine, 8-oxo-guanosine, 2-amino-guanosine, LNA-guanosine, and 2-azido-guanosine. In the mRNA, the GA at the 5' end may have a cap structure of m7(3'OMeG)(5')ppp(5')(2'OMeA). In the mRNA, all U may be substituted with N1-methyl-pseudouridine. In the mRNA, all U may be substituted with N1-methyl-pseudouridine, and a GA at the 5' terminus may have a cap structure of m7(3'OMeG)(5')ppp(5')(2'OMeA).

The 5'-cap may be Cap0, Cap1, or Cap2. In Cap0, the riboses of both the first and second cap-proximal nucleotides of the mRNA include a 2'-hydroxyl group. In Cap1, the riboses of the first and second cap-proximal nucleotides of mRNA include a 2'-methoxy group and a 2'-hydroxyl group, respectively. In Cap2, the riboses of both the first and second cap-proximal nucleotides of the mRNA comprise a 2'-methoxy group.

Providing an RNA having a 5'-cap or a 5'-cap analog may be achieved by in vitro transcription of a DNA template in the presence of the 5'-cap or the 5'-cap analog. In this case, the 5'-cap or the 5'-cap analog may be introduced into the generated RNA strand co-transcriptionally. Alternatively, for example, the RNA may be produced by in vitro transcription and may be produced post-transcriptionally using a capping enzyme, such as a capping enzyme of vaccinia virus.

The microRNA binding sequence may be tumor-specific. The microRNA may be upregulated or downregulated in tumor cells compared to normal cells. The microRNA binding sequence may inhibit a tumor by binding to a tumor-specific miRNA, a seed sequence thereof, or a miRNA target sequence in vivo.

The microRNA may be selected from the group consisting of: miR-340, miR-133a, miR-34b, miR-19, miR-200, miR-15, miR-16, miR-1298, miR-663a, miR-449a, miR-138, miR-126, miR-10a, miR-10b, miR-140-3p, miR-140-5p, miR-204, miR-424, miR-34a, miR-193a-3p, miR-455-5p, miR-455-3p, miR-9, miR-10a, miR-148a, miR-488, miR-196a1, miR-182, miR-96, miR-193b, miR-27a, miR-196b, miR-10b, miR-29b2, miR-23a, miR-107, miR-542-3p, miR-93, miR-365a-4, miR-450a, miR-100, miR-105, miR-363, miR-105, miR-106b, miR-15b, miR-21, miR-376c, miR-93, miR-99b, miR-155, miR-33a, miR-876-3p, miR-362-3p, miR-25, let-7i, miR-423-3p, miR-34b, miR-16-2, miR-29a, miR-30d, miR-320, miR-181c, miR-128a, miR-21, let-7d, miR-450b-5p, miR-371-5p, miR-1245, miR-335, miR-492, miR-874, miR-30b, miR-193a-5p, miR-602, miR-346, miR-663, miR-25, miR-219-5p6, miR-184, miR-135a7, miR-584, miR-665, miR-638, miR-503, miR-628-3p, miR-381, miR-78, miR-92b, miR-149, miR-135b, miR-302d, miR-498, miR-766, miR-1389, miR-623, miR-519c-5p, miR-182, miR-494, miR-129-5p10, miR-513-5p, miR-200b, miR-634, miR-654-5p, miR-518b, miR-658, miR-373, miR-30c-2, miR-130a, miR-557, miR-551a, miR-637, miR-518c, miR-525-5p, miR-596, miR-552, miR-625, miR-183, miR-187, miR-544, miR-891a, miR-519e, miR-933, miR-939, miR-214, miR-671-5p, miR-137, miR-92b, miR-525-3p, miR-19a, and miR-409-5p. An example of the microRNA may be one that is downregulated in tumor cells. The downregulated miR may be miR-34a. However, the differential expression of miRs in cancer cells may vary depending on the type of cancer.

The mRNA may further include a chain terminating nucleoside. The chain-terminating nucleoside may be selected from the group consisting of 3'-deoxyadenosine (cordycepin), 3'-deoxyuridine, 3'-deoxycytosine, 3'-deoxyguanosine, 3'-deoxythymidine, 2',3'-dideoxynucleoside, 2',3'-dideoxyadenosine, 2',3'-dideoxyuridine, 2',3'-dideoxycytosine, 2',3'-dideoxyguanosine, 2',3'-dideoxythymidine, 2'-deoxynucleoside, and O-methylnucleoside. The 3' tail region may include a three-dimensional structure, for example, a stem loop structure.

A second aspect provides a composition for delivering an mRNA molecule or a product expressed therefrom to a subject, including the mRNA molecule as an active ingredient.

A third aspect provides a kit for delivering an mRNA molecule or a product expressed therefrom to a subject, including the mRNA molecule as an active ingredient.

In the second and third aspects, the composition or kit may be for treating cancer. The cancer may be a blood cancer or a solid cancer.

The mRNA molecule may encode a polypeptide. The polypeptide may be any polypeptide having anti-cancer activity. The polypeptide having the above anti-cancer activity is the same as described in the first aspect.

The composition is administered to a subject, and the RNA (e.g., mRNA) may be translated in vivo to produce the polypeptide.

The composition may be contacted with a cell, tissue or organism in an "effective amount".

The effective amount may be determined based on the target tissue, target cells, means of administration, physical characteristics of the polynucleotide (e.g., size and the degree of modified nucleoside content), and other components of the composition.

The composition may be administered in combination with other prophylactic or therapeutic compounds. The prophylactic or therapeutic compound may be, for example, an anti-cancer agent. As used herein, the anti-cancer agent may be any known agent.

The composition may be administered intramuscularly, subcutaneously, intradermally, intranasally, or to the lungs. The composition may be administered locally to a tissue where cancer is present. The composition may be administered locally to a tissue where cells having a high level of cancer-specific miRNA, either intracellularly or extracellularly, are present. The composition may be administered locally to a tissue where cells in which a cancer-specific miRNA is upregulated or downregulated compared to normal cells, either intracellularly or extracellularly, are present.

The composition may include a pharmaceutically acceptable carrier, diluent, or excipient. The polynucleotide in the composition may be formulated or complexed with the carrier, diluent, or excipient. The carrier, diluent, or excipient may be those known in the art.

As used herein, the term "active ingredient" refers to the composition or a polynucleotide included therein, for example, RNA including mRNA. The RNA may, for example, encode a polypeptide having anti-cancer activity.

The relative amounts of the active ingredient, the pharmaceutically acceptable carrier, the excipient, and/or other additional components included in the composition may vary depending on the identity, size, and/or condition of the subject to be treated and the route of administration. For example, the composition may include 0.1% to 100%, for example, 0.5% to 50%, 1.0% to 30%, 5.0% to 80%, or 80% (w/w) or more of the active ingredient.

The composition may be formulated as a nanoparticles. The nanoparticle may be those known for delivering a polynucleotide, such as mRNA, to cells. For example, the nanoparticle may be lipid nanoparticles (LNP). The composition may be formulated as lipid nanoparticles (LNPs) or may be bound to LNPs. The binding includes binding within or on the surface of the LNPs. For example, the composition may be formulated within a lipid polycation complex or may be bound to a lipid polycation complex. The lipid polycation complex is also referred to as a cationic lipid nanoparticle. The polycation may include cationic polypeptides such as MC3, Lipid 319, C12-200, 5A2-SC8, 306Oi10, Moderna Lipid 5, Acuitas A9, SM-102, ALC-0315, Arcturus Lipid 2,2(8,8) 4C CH3, Genevant CL1, polylysine, polyornithine, and/or polyarginine. In addition, the composition may be formulated in or bound to lipid nanoparticles including non-cationic lipids, such as distearoylphosphatidylcholine (DSPC), 1,2-distearoyl-sn-glycero-3-phosphocholine (DSPC), dipalmitoyl phosphatidylcholine (DPPC), sterols such as cholesterol, or dioleoyl phosphatidylethanolamine (DOPE). Furthermore, the composition may be formulated in or bound to lipid nanoparticles including PEG-lipids, such as 1,2-dimyristoyl-rac-glycero-3-methoxypolyethylene glycol-2000 (PEG2000-DMG), 2-[(polyethylene glycol)-2000]-N,N-ditetradecylacetamide (ALC-0159), or polyethylene glycol dimethacrylate (PEG-DMA).

The lipid nanoparticle formulation may include a cationic lipid, a phospholipid, a sterol such as cholesterol, a PEG-lipid, or a combination thereof. For example, the lipid nanoparticle formulation may include a cationic lipid, a phospholipid, a sterol such as cholesterol, and a PEG-lipid. In addition, the lipid nanoparticle may include a PEG-modified lipid, a non-cationic lipid, a sterol, and an ionizable lipid, or a combination thereof. The lipid nanoparticle may include 0.5 to 15 mol% of the PEG-modified lipid, 5 to 25 mol% of the non-cationic lipid, 25 to 55 mol% of the sterol, and 20 to 60 mol% of the ionizable lipid. The PEG-modified lipid may be 1,2-dimyristoyl-sn-glycerol methoxypolyethylene glycol (PEG2000-DMG), the non-cationic lipid may be 1,2-distearoyl-sn-glycero-3-phosphocholine (DSPC), the sterol may be cholesterol, and the ionizable cationic lipid may be Compound 1 having the following structure:

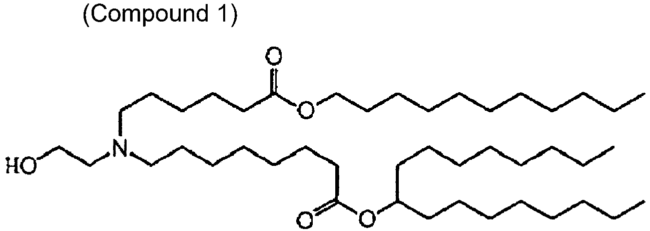

The PEG-modified lipid may be Compound 2 (ALC-0159) having the following structure, the non-cationic lipid may be 1,2-distearoyl-sn-glycero-3-phosphocholine (DSPC), the sterol may be cholesterol, and the ionizable cationic lipid may be Compound 3 (ALC-0315) having the following structure:

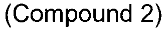

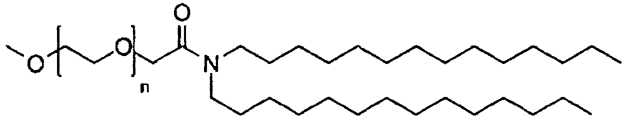

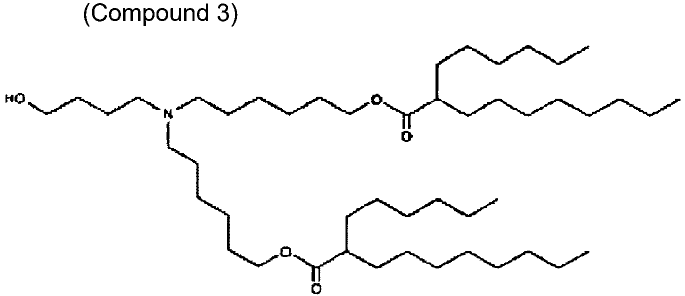

A fourth aspect provides a method of delivering an mRNA or a product expressed therefrom to a subject, including introducing the mRNA molecule into a host cell.

In the method, the host cell may be any cell derived from a subject. The subject may be a mammal, including a human. The cell may be, for example, a stem cell, a germ cell, or a somatic cell. In the method, the host cell may be a cancer cell, an antigen-presenting cell including a dendritic cell, a monocyte, or a macrophage. The introducing may include locally administering to a tissue where cancer is present. The introducing may include locally administering a nucleic acid molecule or an mRNA molecule to a tissue where cells having a high level of cancer-specific miRNA, either intracellularly or extracellularly, are present. The administering may include locally administering a nucleic acid molecule or an mRNA molecule to a tissue where cells in which a cancer-specific miRNA is upregulated or downregulated compared to normal cells, either intracellularly or extracellularly, are present.

The method may include administering the mRNA to a subject. The administering may be parenteral or oral administration. The administering may be intramuscular, subcutaneous, intradermal, intranasal, or intrapulmonary administration. The subject may be a mammal, including a human. The administering may include locally administering to a tissue where cancer is present. The administering may include locally administering a polynucleotide, for example, an mRNA molecule, to a tissue where cells having a high level of cancer-specific miRNA, either intracellularly or extracellularly, are present. The administering may include locally administering a polynucleotide, for example, an mRNA molecule, to a tissue where cells in which a cancer-specific miRNA is upregulated or downregulated compared to normal cells, either intracellularly or extracellularly, are present.

The method may be for inhibiting cancer cell proliferation by producing the polypeptide in a cell. In addition, the method may be for treating a disease in a subject. The polypeptide may be a cancer cell antigen protein.

A sixth aspect provides a DNA molecule encoding the mRNA.

The DNA molecule may include a nucleotide sequence for introducing a transcribable nucleotide sequence and an expression regulatory sequence. The expression regulatory sequence may be a transcriptional regulatory sequence, a translational regulatory sequence, or a sequence involved in the stabilization of RNA. The expression regulatory sequence may be located upstream or downstream of a nucleotide sequence for introducing a transcribable nucleotide sequence. The expression regulatory sequence may be located downstream of the nucleotide sequence for introducing the transcribable nucleotide sequence and upstream of the modified polyadenylation sequence. The nucleotide sequence for introducing the transcribable nucleotide sequence may be a cloning site. The cloning site may be a multiple cloning site. The cloning site may contain a recognition site of a restriction enzyme, a cleavage site, or a combination thereof.

As used herein, the term "transcription" refers to a process in which the genetic code of a DNA sequence is transcribed into RNA. The RNA may then be translated into a protein. The term "transcription" includes *in vitro* transcription. The term "*in vitro* transcription" relates to a process in which RNA, particularly mRNA, is synthesized *in vitro* in a cell-free system. Cloning vectors are applied for the production of transcripts. These cloning vectors are also generally referred to as transcription vectors. The term "vector" includes a transcription vector. The RNA is, for example, *in vitro* transcribed RNA (IVT-RNA) and may be obtained by *in vitro* transcription of a DNA template. A promoter for controlling transcription may be any promoter for any RNA polymerase. A DNA template for *in vitro* transcription may be obtained by cloning a nucleic acid, particularly a cDNA, and may be introduced into an appropriate vector for *in vitro* transcription.

The expression regulatory sequence may be a promoter, a ribosome binding site, a 5'-untranscribed sequence, or a UTR.

As used herein, the term "promoter" or "promoter region" relates to a DNA sequence upstream (5') of a coding sequence of a gene, and refers to controlling the expression of the coding sequence by providing recognition and binding sites for RNA polymerase. The promoter region may further include a recognition or binding site for additional factors involved in regulating the transcription of the gene. The promoter may control the transcription of prokaryotic or eukaryotic genes. The promoter may be "inducible," initiating transcription in response to an inducer, or it may be "constitutive" if transcription is not controlled by an inducer. In the absence of an inducer, the transcription of a gene under the control of the inducible promoter may occur rarely or not at all. In the presence of the inducer, the transcription of the gene under the control of the inducible promoter may increase.

The promoter may be derived from a homologous or heterologous source with respect to the sequence encoding the polypeptide. The promoter may be derived from a mammal including a human. The promoter may be derived from a non-human mammal. The promoter may be derived from a plant, a microorganism or a virus. The microorganism may be yeast or bacteria. The bacteria may be Gram-positive or Gram-negative bacteria. The bacteria may belong to the genus *Escherichia.* The promoter may be a T7, T3, or SP6 promoter. The promoter may be operable in a microorganism of the genus *Escherichia*. The microorganism may be *E. coli*.

In the DNA molecule, the promoter may be a T7 promoter and the microRNA may be microRNA-34a.

In the DNA molecule, the sequence encoding the polypeptide and the modified polyadenylation sequence may be transcribed under the control of a promoter to generate a common transcript. The modified polyadenylation sequence may be located at the 3' end of the common transcript.

As used herein, the term "nucleic acid capable of being transcribed to generate a common transcript" indicates that the nucleic acid sequences are operatively linked to each other such that, when transcribed, an RNA molecule is formed including a transcript in which the nucleic acid sequences are covalently linked to each other. The transcription may be transcription occurring under the control of a promoter, where appropriate after linearization of the nucleic acid molecule, for example, a closed circular nucleic acid molecule, for example, after restriction enzyme cleavage of the nucleic acid molecule. The common transcript may be separated by a sequence located between the nucleic acid sequences, where appropriate.

The DNA molecule may be a closed circular molecule or a linear molecule.

The DNA molecule may further include one or more selected from the group consisting of (i) a reporter gene, (ii) a selectable marker, and (iii) an origin of replication. The nucleic acid molecule may be a vector. The vector may be a cloning vector or an expression vector.

The DNA molecule, after being linearized, may be suitable for *in vitro* transcription of mRNA.

In the DNA molecule, the nucleic acid sequence for introducing the transcribable nucleic acid sequence may be a cloning site. The nucleic acid molecule may include a nucleic acid molecule for introducing a transcribable nucleic acid sequence, including a multiple cloning site.

As used herein, the term "cloning site" refers to a restriction enzyme recognition or cleavage site sequence. The cloning site may include, for example, Hind IIII, Xho I, Nhe I, and Eco RI restriction sites. The nucleic acid molecule may further include a cloning site at a position different from the nucleic acid sequence for introducing the transcribable nucleic acid sequence. The nucleic acid molecule may include a cloning site within the expression regulatory sequence. The nucleic acid molecule may include a cloning site downstream of the modified polyadenylation sequence. The downstream of the modified polyadenylation sequence may be adjacent to the modified polyadenylation sequence or located within 20 nucleotides thereof.

As used herein, the term "expression" is used in its general sense and includes the production of RNA, or RNA and protein. The expression also includes partial expression of a nucleic acid. Additionally, the expression may be transient or stable. With respect to RNA, the term "expression" or "translation" relates to a process in the ribosome of a cell in which a strand of messenger RNA directs the assembly of an amino acid sequence to produce a peptide or protein.

A seventh aspect provides a method of propagating a nucleic acid molecule, including: (i) providing the DNA molecule; and (ii) propagating the nucleic acid molecule in a cell.

In the method, the cell may be a host cell. The term "host cell" refers to any cell into which a foreign nucleic acid may be transformed or transduced. The host cell may include prokaryotic cells (e.g., *E. coli*) or eukaryotic cells (e.g., yeast cells and insect cells). The cells may be an animal, plant or microbial cell. The animal may be a mammal. The mammals include humans, mice, rats, hamsters, pigs, and primates. The cells may be derived from a plurality of tissue cells and may include primary cells and cell lines. The microorganism may be yeast or bacteria. The bacteria may be Gram-positive or Gram-negative bacteria. The bacteria may belong to the genus *Escherichia*. The above cells may be *E. coli*.

In the method, the proliferating may include transforming a cell with the nucleic acid molecule and culturing the transformed cell.

The method may include isolating the nucleic acid molecule from the cell after proliferation.

An eighth aspect provides a method of obtaining RNA, including: (i) propagating a nucleic acid molecule according to the method of the seventh aspect; and (ii) *in vitro* transcribing the nucleic acid molecule as a template into RNA.

The term "*in vitro* transcription" or "RNA *in vitro* transcription" refers to a process in which RNA, including mRNA, is synthesized in a cell-free system, i.e., *in vitro*. Cloning vector DNA, including plasmid DNA vectors, may be applied as a template for the generation of RNA transcripts. These cloning vectors are generally also referred to as transcription vectors. The RNA may be obtained by DNA-dependent *in vitro* transcription of an appropriate DNA template. The DNA template may be a linearized plasmid DNA template. A promoter for controlling RNA *in vitro* transcription may be any promoter for a DNA-dependent RNA polymerase. The DNA-dependent RNA polymerase may be T7 RNA polymerase, T3 RNA polymerase, SP6 RNA polymerase, or a combination thereof. A DNA template for RNA *in vitro* transcription may be obtained by cloning a nucleic acid and introducing it into a vector for RNA *in vitro* transcription. The vector for RNA *in vitro* transcription may be a circular plasmid DNA. The cDNA may be obtained by reverse transcription of mRNA or by chemical synthesis.

A ninth aspect provides a method of obtaining a polypeptide, including: (i) obtaining an mRNA encoding the polypeptide according to the method of the eighth aspect; and (ii) translating the mRNA into the polypeptide.

The method may include, prior to (i), cleaving the nucleic acid molecule.

A tenth aspect provides an RNA molecule obtained by transcribing the nucleic acid molecule of the sixth aspect. The RNA molecule may be an mRNA molecule. The RNA molecule may be an RNA molecule obtained according to the method of the third aspect.

### Advantageous Effects

According to an aspect, the mRNA is relatively stable and has high translation efficiency, and thus may be used to efficiently translate the mRNA.

According to another aspect, the DNA molecule may be used to produce RNA with increased stability.

According to a method of propagating a DNA molecule according to another aspect, the DNA molecule may be efficiently propagated.

According to a method of obtaining RNA according to another aspect, RNA may be obtained efficiently.

According to a method of obtaining a polypeptide according to another aspect, a polypeptide may be obtained efficiently.

A composition or a kit for delivering an mRNA or a product expressed therefrom to a subject according to another aspect may be used for treating cancer.

According to a method of delivering an mRNA or a product expressed therefrom to a subject according to another aspect, the mRNA or the product expressed therefrom may be efficiently delivered to the subject.

### Description of Drawings

FIG. 1 is a view showing a vector obtained by the same processes as in Examples 1 and 2.
FIG. 2 is a view showing a vector including a T7 promoter, a 5' UTR, and a target protein ORF between HindIII and XhoI restriction enzyme sites in the template vector of FIG. 1.
FIG. 3 is a view showing a p53 gene expression vector, p53-polyA-miR34a, which includes a 5' UTR, a p53 gene, a 3' UTR, and a modified polyadenylation sequence including miR34a.
FIG. 4 is a view showing the results of confirming the effect of miRNA-34a on the expression of mRNA introduced into cells through Western blot analysis.
FIG. 5A is a view showing the degree of growth inhibition of a normal cell line, BEAS-2B, by p53-polyA-miR34a mRNA.
FIG. 5B is a view showing the degree of growth inhibition of a lung cancer cell line, H1373, by p53-polyA-miR34a mRNA.
FIG. 5C is a view showing the degree of growth inhibition of a lung cancer cell line, H1299, by p53-polyA-miR34a mRNA.
FIG. 6 is a view showing a template vector for transcription of the constructed OX40L or a fusion protein gene thereof.

### Best Mode

Hereinafter, the present disclosure will be described in more detail through examples. However, these examples are for illustrative purposes to explain the present disclosure, and the scope of the present disclosure is not limited to these examples.

### Example 1: Cloning of 3'UTR Sequence

In this example, the 3'UTR was cloned to increase the expression level and sequence stability of the gene encoding mRNA. The 3' UTR sequence of human cytochrome P450 2E1 was used as the 3' UTR. The 3' UTR was designed to include Xho I and Nhe I restriction enzyme sequences at its 5'-end and 3'-end, respectively, so that it could be used for recombining nucleic acids such as the transcribable nucleic acid sequence or the polyadenylation sequence. The transcribable nucleic acid sequence may be an open reading frame (ORF). The 3'UTR sequence may be linked to the 3'-end of the open reading frame through the XhoI restriction enzyme site at the 5'-end.

A recombinant 3' UTR sequence comprising the Xho I restriction enzyme sequence, the 3' UTR sequence of human cytochrome P450 2E1, and the Nhe I restriction enzyme sequence was synthesized by an in vitro nucleic acid synthesis method. The obtained recombinant 3'UTR sequence is DNA. After treating and cleaving both the recombinant 3' UTR sequence and a template vector with Xho I and Nhe I restriction enzymes, respectively, both sequences were linked using DNA ligase. A pUC57-KanR vector (self-produced) was used as the template vector. The pUC57-KanR vector is a pUC57 vector including Kanamycin as an antibiotic resistance gene. The antibiotic resistance gene is a selectable marker. As a result, a template vector comprising the recombinant 3' UTR sequence was constructed. Table 1 shows the sequence encoding the 3' UTR sequence of human cytochrome P450 2E1.

**[Table 1]**

| SEQ ID NO: | 3'UTR Sequence |
|---|---|
| 1 | GTGTGTGGAGGACACCCTGAACCCCCCGCTTTCAAACAAGTTTTCA AATTGTTTGAGGTCAGGATTTCTCAAACTGATTCCTTTCTTTGCATAT GAGTATTTGAAAATAAATATTTTCCCAGAATATAAATAAATCATCACA TGATTATTTTAACTAT |

In Table 1, the sequence of SEQ ID NO: 1 is the wild-type 3' UTR sequence of the human cytochrome P450 2E1 gene.

### Example 2: Cloning of Consecutive Adenosine Sequence Including miRNA Binding Site

In this example, a recombinant template vector was constructed in which a modified polyadenylation sequence was linked to the 3'-end of the 3' UTR sequence in the recombinant 3' UTR-containing template vector prepared in Example 1. The modified polyadenylation sequence is intended to increase the stability and expression level of a gene encoding mRNA.

The modified polyadenylation sequence includes a nucleotide sequence complementary to miRNA-34a in the middle region of an unmodified 100 nt polyadenylation sequence. Specifically, the modified polyadenylation sequence is one in which the sequences from positions 1 to 22 of the nucleotide sequence complementary to miRNA-34a are introduced between positions 20 and 21 of the unmodified polyadenylation sequence.

In addition, the modified polyadenylation sequence was designed to include Nhe I and Eco RI restriction enzyme sequences at its 5'-end and 3'-end, respectively, so that it could be used for recombination. A recombinant modified polyadenylation sequence including the Nhe I restriction enzyme sequence, the modified polyadenylation sequence, and the Eco RI restriction enzyme sequence was synthesized by an *in vitro* nucleic acid synthesis method. The obtained recombinant sequence is DNA.

The obtained recombinant modified polyadenylation sequence and the recombinant 3' UTR-containing template vector prepared in Example 1 were treated and cleaved with Nhe I and Eco RI restriction enzymes, respectively, and both sequences were linked using DNA ligase to construct a template vector containing a 3' UTR-modified polyadenylation sequence. Table 2 shows the nucleotide sequences of the modified polyadenylation sequence.

**[Table 2]**

| SEQ ID NO: | Poly(A) Tail Sequence |
|---|---|
| 2 | AAAAAAAAAAAAAAAAAAAAACAACCAGCTAAGACACTGCCAAAAAA AAAAAAAAAAAAAAAAAAAAAAAAAAAAAAAAAAAAAAAAAAAAAAAA AAAAAAAAAAAAAAAAAAAAAAAAAAAAAAAAAAAAAAAAAAAAAAA |

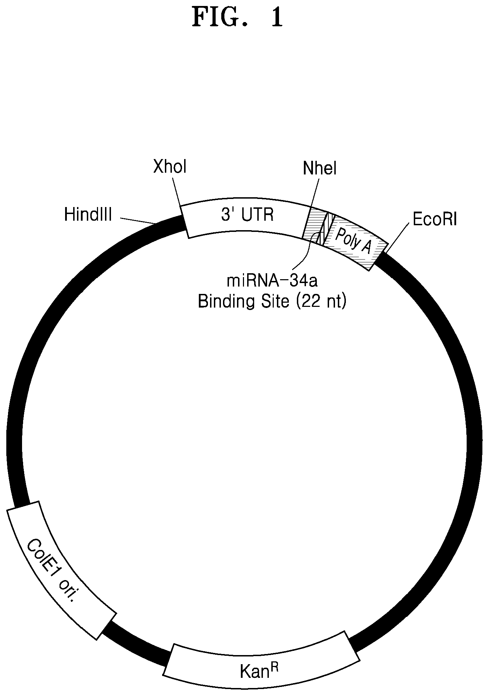

The underlined nucleotides in the sequences listed in Table 2 are sequences complementary to miRNA-34a, i.e., miRNA-34a binding site. FIG. 1 is a view showing a vector obtained by the same processes as in Examples 1 and 2. As shown in FIG. 1, the vector includes a 3' UTR linked to the 5' end of a modified polyadenylation sequence including a miRNA-34a binding sequence in the region of positions 21 to 42 of its nucleotide sequence. The vector includes Hind III and Xho I restriction enzyme recognition sites upstream of the 3' UTR, and thus may be used to introduce a target protein ORF linked to the 3' terminus of a 5' UTR. That is, the vector may be a platform vector used to clone ORF. The 5'UTR-ORF is linked to the T7 promoter upstream thereof. The vector also includes an antibiotic resistance marker (KanR) and an *E. coli* origin of replication (ColE1 ori). The nucleotide sequence of the template vector as shown in FIG. 1 is shown in Table 3.

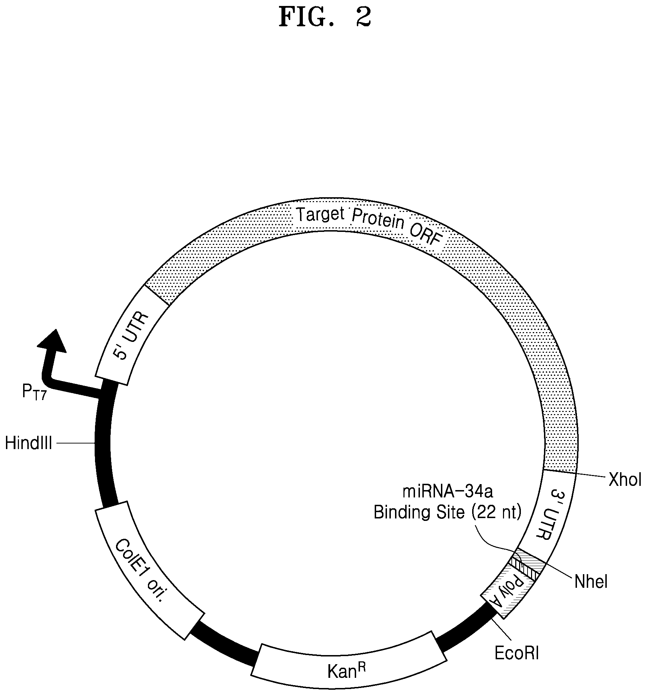

FIG. 2 is a view showing a vector including a T7 promoter, a 5'UTR, and a target protein ORF between HindIII and Xhol restriction enzyme sites in the template vector of FIG. 1.

**[Table 3]**

| SEQ ID NO: | Nucleotide Sequence |
|---|---|
| 3 | AAGCTTCTCGAGgtgtgtggaggacaccctgaaccccccgctttcaaacaagttttcaaattgttt gaggtcaggatttctcaaactgattcctttctttgcatatgagtatttgaaaataaatattttcccagaatataa ataaatcatcacatgattattttaactatgctagcAAAAAAAAAAAAAAAAAAAaacaaccag ctaagacactgccaAAAAAAAAAAAAAAAAAAAAAAAAAAAAAAAAAAAA AAAAAAAAAAAAAAAAAAAAAAAAAAAAAAAAAAAAAAAAAAAAAAAA AAAAAAAAAAAGAAGAGCGAATTCTCAAGACGATAGTTACCGGATAAG GCGCAGCGGTCGGGCTGAACGGGGGGTTCGTGCACACAGCCCAGCT TGGAGCGAACGACCTACACCGAACTGAGATACCTACAGCGTGAGCTA TGAGAAAGCGCCACGCTTCCCGAAGGGAGAAAGGCGGACAGGTATC CGGTAAGCGGCAGGGTCGGAACAGGAGAGCGCACGAGGGAGCTTCC AGGGGGAAACGCCTGGTATCTTTATAGTCCTGTCGGGTTTCGCCACCT CTGACTTGAGCGTCGATTTTTGTGATGCTCGTCAGGGGGGCGGAGCC TATGGAAAAACGCCAGCAACGCGGCCTTTTTACGGTTCCTGGCCTTTT GCTGGCCTTTTGCGAGCCTTTTGCTCACATGTTCTTTCCTGCGTTATC CCCTGATTCTGTGGATAACCGTATTACCGCCTTTGAGTGAGCTGATAC CGCTCGCCGCAGCCGAACGACCGAGCGCAGCGAGTCAGTGAGCGAG GAAGCGGCCCAATACGCAAACCGCCTCTCCCCGCGCGTTGGCCGATT CATTAATGCAGCTGGCACGACAGGTTTCCCGACTGGAAAGCGGGCAG TGAGCGCAACGCAATTAATGTGAGTTAGCTCACTCATTAGGCACCCCA GGCTTTACACTTTATGCTTCCGGCTCGTATGTTGTGTGGAATTGTGAG CGGATAACAATTTCACACAGGAAACAGCTATGACCATGATTACGCC |
| 4 | gtgtgtggaggacaccctgaaccccccgctttcaaacaagttttcaaattgtttgaggtcaggatttctcaa actgattcctttctttgcatatgagtatttgaaaataaatattttcccagaatataaataaatcatcacatgatt |
| | attttaactatAAAAAAAAAAAAAAAAAAAAacaaccagctaagacactgccaGCCACC ACTTCAAGAACTCTGTAGCACCGCCTACATACCTCGCTCTGCTAATCC TGTTACCAGTGGCTGCTGCCAGTGGCGATAAGTCGTGTCTTACCGGG TTGGACTCAAGACGATAGTTACCGGATAAGGCGCAGCGGTCGGGCTG AACGGGGGGTTCGTGCACACAGCCCAGCTTGGAGCGAACGACCTACA CCGAACTGAGATACCTACAGCGTGAGCTATGAGAAAGCGCCACGCTT CCCGAAGGGAGAAAGGCGGACAGGTATCCGGTAAGCGGCAGGGTCG GAACAGGAGAGCGCACGAGGGAGCTTCCAGGGGGAAACGCCTGGTA TCTTTATAGTCCTGTCGGGTTTCGCCACCTCTGACTTGAGCGTCGATT TTTGTGATGCTCGTCAGGGGGGCGGAGCCTATGGAAAAACGCCAGCA ACGCGGCCTTTTTACGGTTCCTGGCCTTTTGCTGGCCTTTTGCGAGCC TTTTGCTCACATGTTCTTTCCTGCGTTATCCCCTGATTCTGTGGATAAC CGTATTACCGCCTTTGAGTGAGCTGATACCGCTCGCCGCAGCCGAAC GACCGAGCGCAGCGAGTCAGTGAGCGAGGAAGCGGCCCAATACGCA AACCGCCTCTCCCCGCGCGTTGGCCGATTCATTAATGCAGCTGGCAC GACAGGTTTCCCGACTGGAAAGCGGGCAGTGAGCGCAACGCAATTAA TGTGAGTTAGCTCACTCATTAGGCACCCCAGGCTTTACACTTTATGCTT CCGGCTCGTATGTTGTGTGGAATTGTGAGCGGATAACAATTTCACACA GGAAACAGCTATGACCATGATTACGCC |

In the sequence of the template vector of SEQ ID NO: 3 described in Table 3, the nucleotides indicated by underlined uppercase letters are the Hind III restriction enzyme recognition sequence. In the sequence of the template vector of SEQ ID NO: 3 described in Table 3, the nucleotides indicated by bold uppercase letters are the Xho I restriction enzyme recognition sequence. In the sequence of the template vector of SEQ ID NO: 3 described in Table 3, the nucleotides indicated by lowercase letters are the wild-type 3' UTR sequence of human cytochrome P450 2E1 (SEQ ID NO: 1).

In the sequence of the template vector of SEQ ID NO: 3 described in Table 3, the nucleotides indicated by bold lowercase letters are the Nhe I restriction enzyme recognition sequence.

In the sequence of the template vector of SEQ ID NO: 3 described in Table 3, the nucleotides indicated by underlined lowercase letters are the miRNA-34a binding sequence complementary to miRNA-34a.

In the sequence of the template vector of SEQ ID NO: 3 described in Table 3, the nucleotides indicated by italicized uppercase letters are the Eco RI restriction enzyme recognition sequence.

SEQ ID NO: 4 in Table 3 is a sequence of SEQ ID NO: 3 excluding the restriction enzyme recognition sites.

### Example 3: Construction of p53 mRNA Transcription Vector

In this example, a template vector to be used for p53 mRNA transcription was constructed using the vector platform as shown in FIG. 1 prepared in Example 2.

### Example 3-1. Cloning of 5' UTR Sequence and Wild-type p53 Gene Sequence

A recombinant gene including a 5' restriction enzyme recognition site, a T7 promoter sequence, a 5' UTR, an open reading frame (ORF), and a 3' restriction enzyme recognition site was synthesized by an *in vitro* nucleic acid synthesis method. The 5' restriction enzyme recognition site and the 3' restriction enzyme recognition site are Hind III and Xho I restriction enzyme sequences, respectively. The 5' UTR is the sequence indicated in bold in Table 4 below. The open reading frame (ORF) is a nucleotide sequence encoding human wild-type p53. As a result, a recombinant sequence was obtained in which the Hind III recognition site, the T7 promoter sequence, the 5' UTR, the p53 ORF, and the Xho I recognition site were linked.

The obtained recombinant sequence and the template vector of FIG. 1 prepared in Example 2 were treated and cleaved with the restriction enzymes Hind III and Xho I, respectively, and both sequences were linked using DNA ligase. As a result, a transcription vector including the 5' UTR, the wild-type p53 ORF, the cytochrome P450 2E1 3' UTR, and the modified polyadenylation sequence was constructed (hereinafter referred to as "p53-polyA-miR34a"). As a control, a vector in which the modified polyadenylation sequence in the p53-polyA-miR34a was substituted with an unmodified poly(A) including 100 As was used (hereinafter referred to as "p53-polyA").

Table 4 shows the nucleotides of the recombinant sequence in which the Hind III recognition site, the T7 promoter sequence, the 5' UTR, the p53 ORF, and the Xho I recognition site are linked. The Hind III and Xho I recognition sites were attached to both ends from which the linked recombinant sequence was obtained.

**[Table 4]**

| SEQ ID NO: | 5' UTR and Wild-type p53 Gene Sequence |
|---|---|
| 5 | TAATACGACTCACTATA |
| 6 | AGTGCTCCCCATCCAACTAAACTGTCCCTCTGTCCGAACTAAACTGCAC TGTCCCAGCACC |
| 7 | TCCGCGAATTGAACGAGGCCCTGGAGTTGAAAGACGCTCAAGCCGGAAA GGAACCAGGTGGGTCTAGGGCCCACTCAAGCCATCTGAAGAGTAAGAAA GGCCAGTCCACTTCCAGGCACAAAAAGTTAATGTTCAAGACGGAGGGTC CTGACTCTGAC |

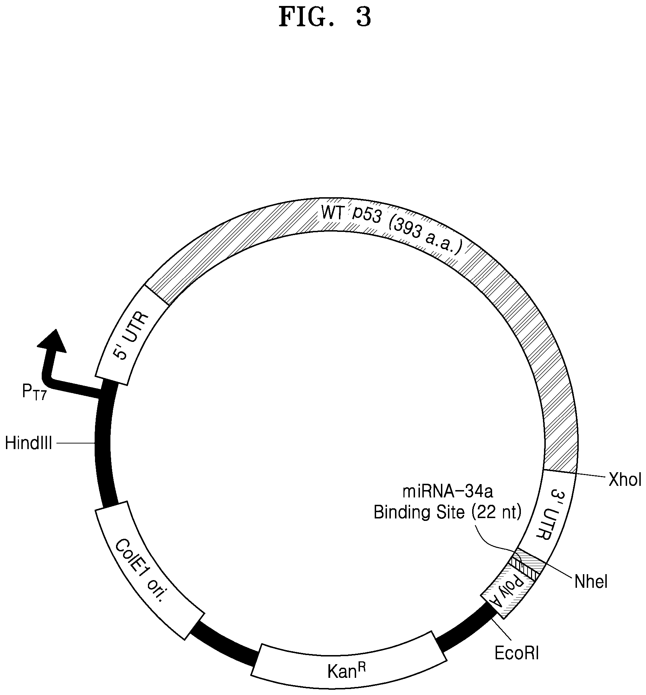

In the sequence described in Table 4, the underlined nucleotides of SEQ ID NO: 5 refer to the T7 promoter portion involved in the initiation of mRNA transcription. In addition, the bold nucleotides of SEQ ID NO: 6 are the 5' UTR sequence. Furthermore, SEQ ID NO: 7 is a nucleotide sequence encoding the wild-type p53 protein as a nucleotide sequence downstream of the 5' UTR. FIG. 3 is a view showing a vector for p53 gene expression, p53-polyA-miR34a, including a 5' UTR, a p53 gene, a 3' UTR, and a modified polyadenylation sequence including miR34a. Table 5 shows the nucleotide sequence of each element among products obtained by *in vitro* transcription of the obtained transcription vector. Table 6 shows the amino acid sequence encoded by the p53 gene included in the obtained transcription vector.

**[Table 5]**

| SEQ ID NO: | Region | mRNA Sequence |
|---|---|---|
| 8 | 5'UTR | AGUGCUCCCCAUCCAACUAAACUGUCCCUCUGUCC GAACUAAACUGCACUGUCCCAGCACC |
| 9 | Wild-type p53 Protein | UCCGCGAAUUGAACGAGGCCCUGGAGUUGAAAGAC GCUCAAGCCGGAAAGGAACCAGGUGGGUCUAGGGC CCACUCAAGCCAUCUGAAGAGUAAGAAAGGCCAGU CCACUUCCAGGCACAAAAAGUUAAUGUUCAAGACGG AGGGUCCUGACUCUGACCUCGAG |
| 10 | 3'UTR | GUGUGUGGAGGACACCCUGAACCCCCCGCUUUCAA ACAAGUUUUCAAAUUGUUUGAGGUCAGGAUUUCUC AAACUGAUUCCUUUCUUUGCAUAUGAGUAUUUGAAA AUAAAUAUUUUCCCAGAAUAUAAAUAAAUCAUCACA UGAUUAUUUUAACUAUGCUAGC |
| 11 | Poly(A) Tail | AAAAAAAAAAAAAAAAAAAAAAACAACCAGCUAAGACAC UGCCAAAAAAAAAAAAAAAAAAAAAAAAAAAAAAAAAAAAA AAAAAAAAAAAAAAAAAAAAAAAAAAAAAAAAAAAAAAAA AAAAAAAAAAAAAAAAAAAAAAAAAAAAAAAAGAAGAGC GAAUU |
| 12 | Wild-type p53 Protein | UCCGCGAAUUGAACGAGGCCCUGGAGUUGAAAGAC GCUCAAGCCGGAAAGGAACCAGGUGGGUCUAGGGC CCACUCAAGCCAUCUGAAGAGUAAGAAAGGCCAGU CCACUUCCAGGCACAAAAAGUUAAUGUUCAAGACGG AGGGUCCUGACUCUGAC |
| 13 | 3'UTR | GUGUGUGGAGGACACCCUGAACCCCCCGCUUUCAA ACAAGUUUUCAAAUUGUUUGAGGUCAGGAUUUCUC AAACUGAUUCCUUUCUUUGCAUAUGAGUAUUUGAAA AUAAAUAUUUUCCCAGAAUAUAAAUAAAUCAUCACA UGAUUAUUUUAACUAU |
| 14 | Poly(A) Tail | AAAAAAAAAAAAAAAAAAAAAACAACCAGCUAAGACAC UGCCAAAAAAAAAAAAAAAAAAAAAAAAAAAAAAAAAAAA AAAAAAAAAAAAAAAAAAAAAAAAAAAAAAAAAAAAAAAA AAAAAAAAAAAAAAAAAAAAAAAAAAAAAA |

In the wild-type p53 protein region of SEQ ID NO: 9 described in Table 5, the underlined nucleotides are the Xho I restriction enzyme recognition sequence. In the 3' UTR region of SEQ ID NO: 10 described in Table 5, the underlined nucleotides are the Nhe I restriction enzyme recognition sequence. In the poly(A) tail region of SEQ ID NO: 11 described in Table 5, the underlined nucleotides are the Sap I restriction enzyme recognition sequence. In the poly(A) tail region of SEQ ID NO: 11 described in Table 5, the bold nucleotides are a part of the Eco RI restriction enzyme recognition sequence.

SEQ ID NOs: 12 to 14 described in Table 5 are sequences of SEQ ID NOs: 9 to 11, respectively, excluding the restriction enzyme recognition portions.

**[Table 6]**

| SEQ ID NO: | Wild-type p53 Protein Amino Acid Sequence |
|---|---|
| 15 | |

In Table 6, SEQ ID NO: 15 is the amino acid sequence obtained by the translation of SEQ ID NO: 12.

### Example 3-2. Confirmation of p53 mRNA Expressionin H1299 Cells

An *in vitro* transcription reaction was performed by incubating a mixture containing the p53-polyA-miR34a vector and the p53-polyA vector prepared in Example 3-1, respectively, to obtain p53-polyA-miR34a mRNA and p53-polyA mRNA. The p53-polyA-miR34a mRNA includes the 5' UTR, p53 ORF, 3' UTR, and the polyA-miR34a of SEQ ID NOs: 8, 9, 10, and 11

The obtained p53-polyA-miR34a mRNA or p53-polyA mRNA, and miRNA-34a were delivered to H1299 cells, and expression thereof was confirmed. A synthetic miRNA-34a was used. The H1299 cells are a human non-small cell lung carcinoma cell line derived from a lymph node. H1299 cells do not express the p53 protein due to a homozygous partial deletion of the p53 gene (hereinafter referred to as "p53-null lung cancer cells").

Specifically, H1299 cells were seeded in each well of a 6-well plate at a density of 3x105 cells/2 mL medium/well and incubated at 37 °C for 24 hours. RPMI 1640 (Gibco, Cat No. 22400-089) was used as the medium. Next, the p53-polyA-miR34a mRNA or p53-polyA mRNA was mixed with Lipofectamine^{™} MessengerMAX^{™} Transfection Reagent (Thermo Scientific^{™}, Cat No. LMRNA001) at a ratio of 1 µL per 1 µg of mRNA and incubated for 20 minutes. 1 µg of the obtained reaction product and miRNA-34a were added to each well of the 6-well plate. The miRNA-34a was added at concentrations of 0 nM, 10 nM, and 25 nM, respectively. The reaction mixture was cultured at 37 °C for 24 hours. After completion of the culture, lysis buffer, M-PER^{™} (Mammalian Protein Extraction Reagent) (Thermo Scientific^{™}, Cat No. 78501), was added to each well in an amount of 100 µL/well to lyse the cells and extract proteins, followed by BCA protein quantification using a Pierce^{™} BCA Protein Assay Kit (Thermo Scientific^{™}, Cat No. 23225). The obtained samples were diluted with lysis buffer and a 5x reducing dye (10% SDS, 0.5% bromophenol blue, 50% glycerol, 0.5% 2-mercaptoethanol, and 250 mM Tris, pH 6.8) to equalize the protein concentration. Western blot analysis was performed on the obtained diluted samples using an anti-p53 monoclonal antibody (CST, Cat No. #2527) and an anti-β-actin antibody (Abcam, Cat No. ab8227).

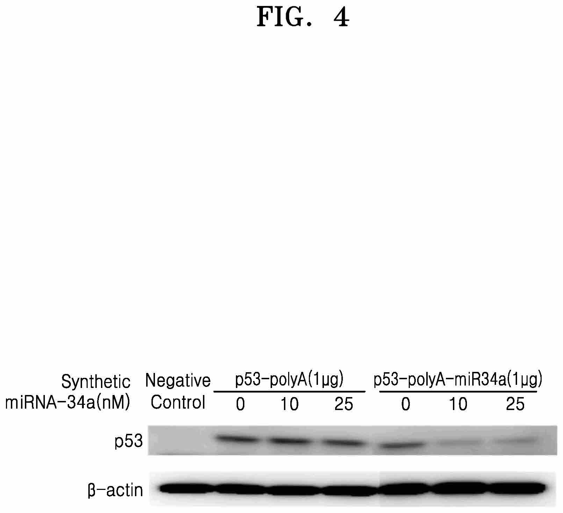

FIG. 4 is a view showing the results of confirming the effect of miRNA-34a on the expression of mRNA introduced into cells through Western blot analysis. As shown in FIG. 4, p53 expression was significantly decreased in p53-polyA-miR34a mRNA compared to p53-polyA mRNA. This indicates that the expression of p53 is decreased by the modified poly(A) including the miR34a binding site.

Specifically, since the p53-polyA mRNA does not have a miRNA-34a binding site at the poly(A) position, there was no difference in p53 expression even when the concentration of the synthetic miRNA-34a increased. On the other hand, the p53-polyA-miR34a mRNA possesses a site where miRNA-34a may bind at the poly(A) position, and thus the expression of p53 decreased in proportion to the concentration of the added synthetic miRNA-34a. This indicates that miRNA-34a normally binds to the miRNA-34a binding site inserted at the poly(A) position to regulate p53 expression. However, the claimed invention is not limited by a specific mechanism.

### Example 3-3. Confirmation of Cell Growth Inhibition in BEAS-2B, H1373, and H1299 Cells

An *in vitro* transcription reaction was performed by incubating a mixture containing the p53-polyA-miR34a vector prepared in Example 3-1 to obtain p53-polyA-miR34a mRNA. The p53-polyA-miR34a mRNA includes the 5' UTR, the p53 ORF, the 3' UTR, and the polyA-miR34a of SEQ ID NOs: 8, 9, 10, and 11.

The obtained p53-polyA-miR34a mRNA was delivered to normal cells and cancer cells, and the cell growth inhibitory activity was confirmed. BEAS-2B, a human bronchial epithelial cell line, was used as the normal cell line, and H1373 and H1299 cells were used as the cancer cell lines. The H1373 cell line is a lung adenocarcinoma cell line in which the p53 gene is mutated and its expression is not detected by Western blot.

Specifically, BEAS-2B, H1373, and H1299 cells were seeded in each well of a 96-well plate at densities of 3x103 cells/200 µL medium/well, 3x103 cells/200 µL medium/well, and 5x103 cells/200 µL medium/well, respectively, and incubated at 37 °C for 24 hours. For the culture medium, BEBM (Lonza, Cat No. CC-3171) was used for BEAS-2B cells, and RPMI 1640 (Gibco, Cat No. 22400-089) was used for H1373 and H1299 cells.

Next, the p53-polyA-miR34a mRNA and Lipofectamine^{™} MessengerMAX^{™} Transfection Reagent (Thermo Scientific^{™}, Cat No. LMRNA001) were mixed at a ratio of 1 µL per 1 µg of p53-polyA-miR34a mRNA and incubated for 20 minutes. 0.2 µL of the obtained reaction product was added to each well of the 96-well plate. The p53-polyA-miR34a mRNA was added at eight concentrations, starting from 4 µg/mL with 4-fold serial dilutions. After culturing the cells in the medium contained in each well of the resulting plate at 37 °C for 3 days, cell viability was measured using a WST-8 cell viability assay kit (BIOMAX, Cat No. QM5000).

Upon completion of the culture, the WST-8 reagent was mixed with the cell culture medium at a ratio of 1:10 and allowed to react for 2 hours. Subsequently, the absorbance at a wavelength of 450 nm was measured, and the GI₅₀ value was derived using Graphpad PrismTM software. The GI₅₀ represents the half maximal growth inhibition concentration.

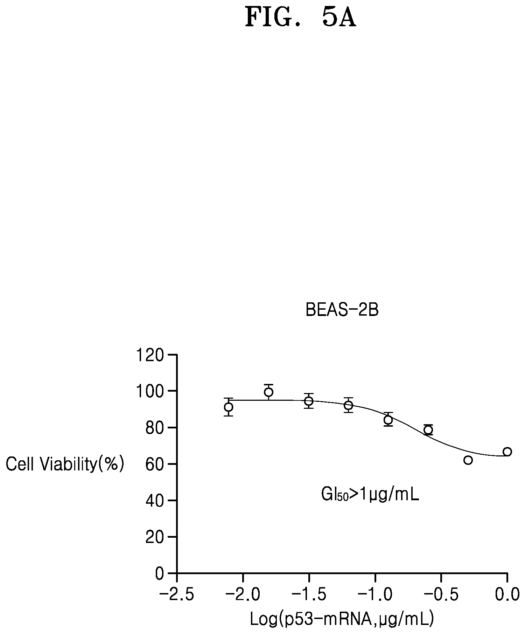

FIG. 5A is a view showing the degree of growth inhibition of the normal cell line, BEAS-2B, by p53-polyA-miR34a mRNA.

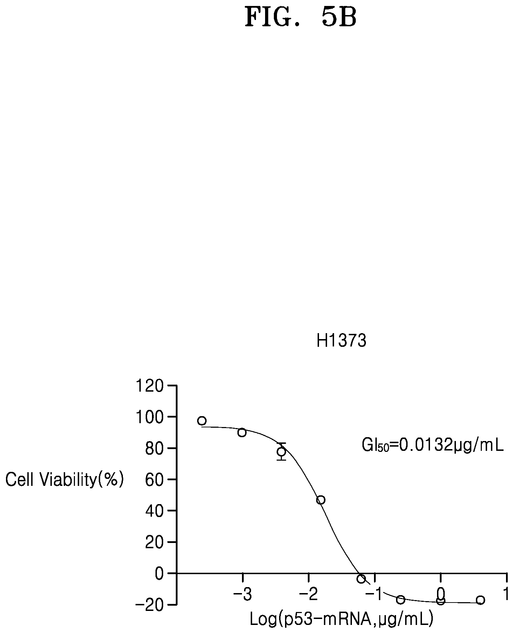

FIG. 5B is a view showing the degree of growth inhibition of the lung cancer cell line, H1373, by p53-polyA-miR34a mRNA.

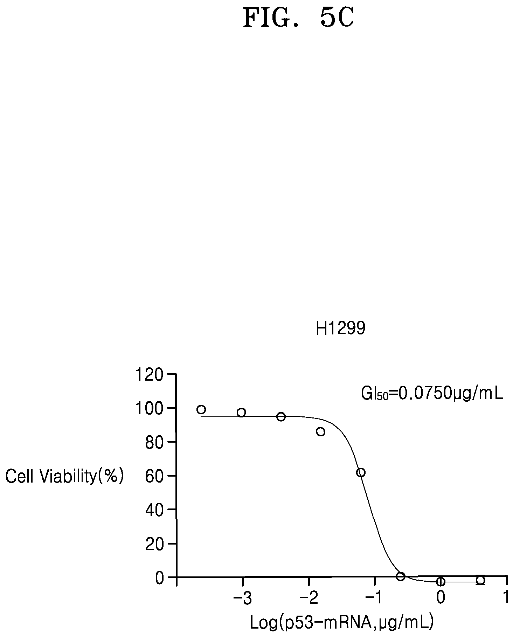

FIG. 5C is a view showing the degree of growth inhibition of the lung cancer cell line, H1299, by p53-polyA-miR34a mRNA.

As shown in FIGS. 5A, 5B, and 5C, the mRNA into which the miRNA binding site was introduced exhibited almost no growth inhibition in normal cells, while specifically inhibiting cell growth in cancer cells.

### Example 4. Construction of OX40L mRNA Transcription Vector

In this example, a template vector to be used for OX40L mRNA transcription was constructed using the vector platform (FIG. 1) prepared in Example 2.

A recombinant gene including a 5' restriction enzyme recognition site, a T7 promoter sequence, a 5' UTR, an open reading frame (ORF), and a 3' restriction enzyme recognition site was synthesized by an in vitro nucleic acid synthesis method. The 5' restriction enzyme recognition site and the 3' restriction enzyme recognition site are Hind III and Xho I restriction enzyme sequences, respectively. The open reading frame is a sequence encoding wild-type OX40L, a fusion protein of OX40L-alpha helix region of MBL-ferritin, or a fusion protein of MBL-OX40L, respectively. As for the ferritin, a heavy chain region of wild-type ferritin was used. MBL refers to mannan-binding lectin, and a sequence from which a carbohydrate recognition domain (CRD) has been removed was used.

As a result, a recombinant sequence was obtained in which the Hind III recognition site, the T7 promoter sequence, the 5' UTR, each of the aforementioned ORFs, and the Xho I recognition site were linked. The fusion protein includes a linker sequence between each fusion partner. The linker sequence is a G4S, i.e. GGGGS sequence.

Next, the obtained recombinant sequence and the template vector of FIG. 1 prepared in Example 2 were treated and cleaved with the restriction enzymes Hind III and Xho I, respectively, and both sequences were linked using DNA ligase.

As a result, a transcription vector including the 5' UTR, each of the aforementioned ORFs, the cytochrome P450 2E1 3' UTR, and the modified polyadenylation sequence was constructed. Table 7 shows the nucleotides of the recombinant sequence in which the T7 promoter sequence, the 5' UTR, the OX40L ORF, the ORF of a fusion protein of OX40L-alpha helix region of MBL-ferritin, or the ORF of a fusion protein of MBL-OX40L are linked. The Hind III recognition site and Xho I recognition site sequences are not included in the 5' UTR and the ORF, respectively, and were used only for cloning.

**[Table 7]**

| SEQ ID NO: | Region | Nucleotide Sequence |
|---|---|---|
| 5 | T7 Promoter | TAATACGACTCACTATA |
| 6 | 5'UTR | AGTGCTCCCCATCCAACTAAACTGTCCCTCTGTCCGA ACTAAACTGCACTGTCCCAGCACC |
| 16 | Signal Sequence | ATGACAAGGTTGACGGTCCTGGCATTGCTTGCCGGG CTTTTGGCGAGTTCTCGGGCG |
| 17 | Extracellular Domain of OX40L | AGCAGTTCACCTGCAAAGGACCCCCCAATCCAGCGA CTGAGAGGCGCAGTCACCCGGTGTGAAGATGGACA GCTTTTTATATCATCTTACAAGAATGAGTATCAGACTA TGGAGGTTCAAAATAACTCCGTGGTTATAAAGTGCGA CGGGCTTTACATCATCTATCTGAAGGGGTCCTTTTTC CAGGAGGTCAAGATAGATTTGCACTTCAGGGAAGAC CATAACCCCATTTCTATTCCTATGCTTAATGACGGAC GACGGATCGTATTTACTGTTGTGGCTTCTCTCGCTTT TAAGGACAAGGTCTACCTTACCGTGAATGCCCCAGA CACACTTTGCGAGCACCTCCAGATCAACGACGGAGA GCTTATCGTTGTTCAACTTACACCAGGTTATTGCGCT CCAGAGGGGAGCTATCACTCCACCGTCAACCAGGTA CCCCTT |
| 18 | G4S Linker*2 | GGTGGAGGCGGTTCAGGCGGAGGTGGCTCT |
| 19 | Alpha Helix of MBL | AGCGAGAGAAAGGCCCTTCAGACAGAAATGGCACGC ATAAAAAAATGGCTTACATTCAGT |
| 31 | G4S Linker | GGTGGAGGCGGTTCA |
| 20 | Ferritin | CAGGACTCCGAAGCAGCAATAAACCGACAAATAAATC TGGAGTTGTACGCATCCTACGTTTACCTGTCCATGTC TTACTACTTCGATAGAGACGATGTCGCCCTCAAAAAC TTCGCCAAGTATTTCCTGCATCAGTCCCATGAAGAGA GAGAGCACGCAGAGAAACTGATGAAACTTCAGAACC AACGGGGGGGCAGGATCTTTCTGCAAGACATTAAGA AGCCTGATTGCGATGACTGGGAATCTGGACTTAATG CCATGGAGTGTGCCCTGCACCTCGAAAAAAATGTGA ACCAGAGCCTGTTGGAATTGCATAAGCTGGCAACCG ATAAGAACGATCCACATCTTTGCGATTTCATCGAAAC CCACTATCTCAATGAACAAGTGAAGGCAATTAAGGAG CTGGGCGATCATGTAACAAATTTGCGAAAAATGGGTG CC |
| 5 | T7 Promoter | TAATACGACTCACTATA |
| 6 | 5'UTR | AGTGCTCCCCATCCAACTAAACTGTCCCTCTGTCCGA ACTAAACTGCACTGTCCCAGCACC |
| 16 | Signal Sequence | ATGACAAGGTTGACGGTCCTGGCATTGCTTGCCGGG CTTTTGGCGAGTTCTCGGGCG |
| 21 | MBL | TCTGGCTCTCAGACTTGTGAAGATACCCTTAAGACTT GCAGTGTCATAGCCTGTGGGCGAGACGGGCGCGAT GGACCCAAGGGCGAAAAAGGAGAACCAGGGCAAGG GCTCAGAGGACTGCAAGGTCCACCTGGTAAATTGGG GCCACCTGGAAGCGTGGGGTCCCCAGGAAGTCCAG GCCCAAAAGGTCAGAAAGGGGACCACGGCGATAATC GAGCCATTGAGGAAAAACTCGCAAATATGGAGGCCG AAATTAGGATTCTGAAGAGTAAGTTGCAGCTGACCAA CAAGCTCCATGCTTTTAGCATGGGC |
| 18 | G4S Linker*2 | GGTGGAGGCGGTTCAGGCGGAGGTGGCTCT |
| 17 | Extracellular Domain of OX40L | AGCAGTTCACCTGCAAAGGACCCCCCAATCCAGCGA CTGAGAGGCGCAGTCACCCGGTGTGAAGATGGACA GCTTTTTATATCATCTTACAAGAATGAGTATCAGACTA TGGAGGTTCAAAATAACTCCGTGGTTATAAAGTGCGA CGGGCTTTACATCATCTATCTGAAGGGGTCCTTTTTC CAGGAGGTCAAGATAGATTTGCACTTCAGGGAAGAC CATAACCCCATTTCTATTCCTATGCTTAATGACGGAC GACGGATCGTATTTACTGTTGTGGCTTCTCTCGCTTT TAAGGACAAGGTCTACCTTACCGTGAATGCCCCAGA CACACTTTGCGAGCACCTCCAGATCAACGACGGAGA GCTTATCGTTGTTCAACTTACACCAGGTTATTGCGCT CCAGAGGGGAGCTATCACTCCACCGTCAACCAGGTA CCCCTT |

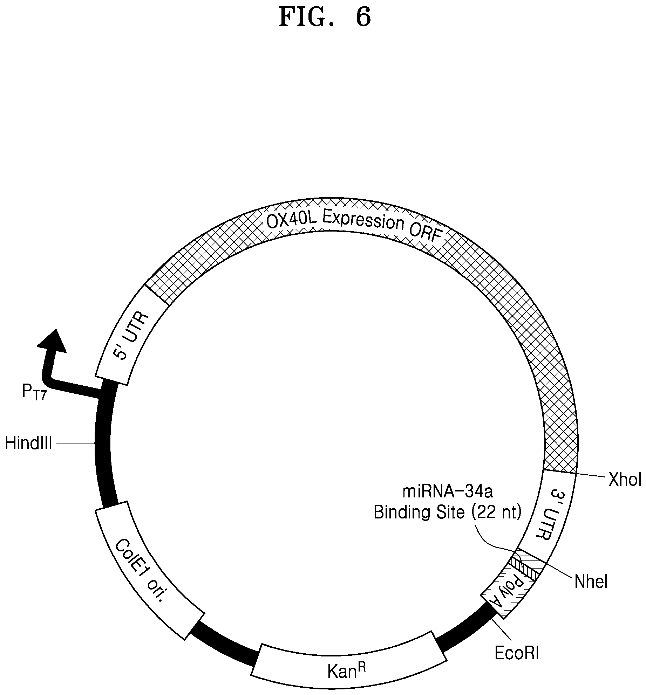

FIG. 6 is a diagram showing a template vector for the transcription of the constructed OX40L or a fusion protein gene thereof. As shown in FIG. 6, the template vector includes a promoter-5' UTR-OX40L ORF-CYP450 2E1 3' UTR-modified polyadenylation sequence containing a miRNA-34a binding sequence. In addition, the template vector includes a ColE1 ori and a KanR gene. Table 8 shows the nucleotide sequence of each element among products obtained by in vitro transcription of the obtained transcription vector. Table 9 shows the amino acid sequence encoded by each ORF included in the obtained transcription vector.

**[Table 8]**

| Mat eria l | SEQ ID NO | Region | mRNA Sequence |
|---|---|---|---|
| 1 | 8 | 5'UTR | AGUGCUCCCCAUCCAACUAAACUGUCCCUCUGUCC GAACUAAACUGCACUGUCCCAGCACC |
| | 22 | Signal Sequence | AUGACAAGGUUGACGGUCCUGGCAUUGCUUGCCG GGCUUUUGGCGAGUUCUCGGGCG |
| | 23 | Extracellular Domain of OX40L | AGCAGUUCACCUGCAAAGGACCCCCCAAUCCAGCG ACUGAGAGGCGCAGUCACCCGGUGUGAAGAUGGAC AGCUUUUUAUAUCAUCUUACAAGAAUGAGUAUCAGA CUAUGGAGGUUCAAAAUAACUCCGUGGUUAUAAAG UGCGACGGGCUUUACAUCAUCUAUCUGAAGGGGUC CUUUUUCCAGGAGGUCAAGAUAGAUUUGCACUUCA GGGAAGACCAUAACCCCAUUUCUAUUCCUAUGCUU AAUGACGGACGACGGAUCGUAUUUACUGUUGUGGC UUCUCUCGCUUUUAAGGACAAGGUCUACCUUACCG UGAAUGCCCCAGACACACUUUGCGAGCACCUCCAG AUCAACGACGGAGAGCUUAUCGUUGUUCAACUUAC ACCAGGUUAUUGCGCUCCAGAGGGGAGCUAUCACU CCACCGUCAACCAGGUACCCCUU |
| | 24 | G4S Linker*2 | GGUGGAGGCGGUUCAGGCGGAGGUGGCUCU |
| | 25 | Alpha Helix of MBL | AGCGAGAGAAAGGCCCUUCAGACAGAAAUGGCACG CAUAAAAAAAUGGCUUACAUUCAGU |
| | 32 | G4S Linker | GGUGGAGGCGGUUCA |
| | 26 | Ferritin | CAGGACUCCGAAGCAGCAAUAAACCGACAAAUAAAU CUGGAGUUGUACGCAUCCUACGUUUACCUGUCCAU GUCUUACUACUUCGAUAGAGACGAUGUCGCCCUCA AAAACUUCGCCAAGUAUUUCCUGCAUCAGUCCCAU GAAGAGAGAGAGCACGCAGAGAAACUGAUGAAACU UCAGAACCAACGGGGGGGCAGGAUCUUUCUGCAAG ACAUUAAGAAGCCUGAUUGCGAUGACUGGGAAUCU GGACUUAAUGCCAUGGAGUGUGCCCUGCACCUCGA AAAAAAUGUGAACCAGAGCCUGUUGGAAUUGCAUA AGCUGGCAACCGAUAAGAACGAUCCACAUCUUUGC GAUUUCAUCGAAACCCACUAUCUCAAUGAACAAGUG AAGGCAAUUAAGGAGCUGGGCGAUCAUGUAACAAA UUUGCGAAAAAUGGGUGCC |
| | 27 | 3'UTR | CUCGAGGUGUGUGGAGGACACCCUGAACCCCCCGC UUUCAAACAAGUUUUCAAAUUGUUUGAGGUCAGGA UUUCUCAAACUGAUUCCUUUCUUUGCAUAUGAGUA UUUGAAAAUAAAUAUUUUCCCAGAAUAUAAAUAAAU CAUCACAUGAUUAUUUUAACUAUGCUAGC |
| | 11 | Poly(A) Tail | AAAAAAAAAAAAAAAAAAAAACAACCAGCUAAGACAC UGCCAAAAAAAAAAAAAAAAAAAAAAAAAAAAAAAAA AAAAAAAAAAAAAAAAAAAAAAAAAAAAAAAAAAAAAA AAAAAAAAAAAAAAAAAAAAAAAAAAAAAAAGAAGAG CGAAUU |
| 2 | 8 | 5'UTR | AGUGCUCCCCAUCCAACUAAACUGUCCCUCUGUCC GAACUAAACUGCACUGUCCCAGCACC |
| | 22 | Signal Sequence | AUGACAAGGUUGACGGUCCUGGCAUUGCUUGCCG GGCUUUUGGCGAGUUCUCGGGCG |
| | 28 | MBL | UCUGGCUCUCAGACUUGUGAAGAUACCCUUAAGAC UUGCAGUGUCAUAGCCUGUGGGCGAGACGGGCGC GAUGGACCCAAGGGCGAAAAAGGAGAACCAGGGCA AGGGCUCAGAGGACUGCAAGGUCCACCUGGUAAAU UGGGGCCACCUGGAAGCGUGGGGUCCCCAGGAAG UCCAGGCCCAAAAGGUCAGAAAGGGGACCACGGCG AUAAUCGAGCCAUUGAGGAAAAACUCGCAAAUAUG GAGGCCGAAAUUAGGAUUCUGAAGAGUAAGUUGCA GCUGACCAACAAGCUCCAUGCUUUUAGCAUGGGC |
| | 24 | G4S Linker*2 | GGUGGAGGCGGUUCAGGCGGAGGUGGCUCU |
| | 23 | Extracellular domain of OX40L | AGCAGUUCACCUGCAAAGGACCCCCCAAUCCAGCG ACUGAGAGGCGCAGUCACCCGGUGUGAAGAUGGAC AGCUUUUUAUAUCAUCUUACAAGAAUGAGUAUCAGA CUAUGGAGGUUCAAAAUAACUCCGUGGUUAUAAAG UGCGACGGGCUUUACAUCAUCUAUCUGAAGGGGUC CUUUUUCCAGGAGGUCAAGAUAGAUUUGCACUUCA GGGAAGACCAUAACCCCAUUUCUAUUCCUAUGCUU AAUGACGGACGACGGAUCGUAUUUACUGUUGUGGC UUCUCUCGCUUUUAAGGACAAGGUCUACCUUACCG UGAAUGCCCCAGACACACUUUGCGAGCACCUCCAG AUCAACGACGGAGAGCUUAUCGUUGUUCAACUUAC ACCAGGUUAUUGCGCUCCAGAGGGGAGCUAUCACU CCACCGUCAACCAGGUACCCCUU |
| | 27 | 3'UTR | CUCGAGGUGUGUGGAGGACACCCUGAACCCCCCGC UUUCAAACAAGUUUUCAAAUUGUUUGAGGUCAGGA UUUCUCAAACUGAUUCCUUUCUUUGCAUAUGAGUA UUUGAAAAUAAAUAUUUUCCCAGAAUAUAAAUAAAU CAUCACAUGAUUAUUUUAACUAUGCUAGC |
| | 11 | Poly(A) Tail | AAAAAAAAAAAAAAAAAAAAAAACAACCAGCUAAGACAC UGCCAAAAAAAAAAAAAAAAAAAAAAAAAAAAAAAAAAA AAAAAAAAAAAAAAAAAAAAAAAAAAAAAAAAAAAAAAAA AAAAAAAAAAAAAAAAAAAAAAAAAAAAAAAAAGAAGAG CGAAUU |
| 3 | 8 | 5'UTR | AGUGCUCCCCAUCCAACUAAACUGUCCCUCUGUCC GAACUAAACUGCACUGUCCCAGCACC |
| | 22 | Signal Sequence | AUGACAAGGUUGACGGUCCUGGCAUUGCUUGCCG GGCUUUUGGCGAGUUCUCGGGCG |
| | 23 | Extracellular Domain of OX40L | AGCAGUUCACCUGCAAAGGACCCCCCAAUCCAGCG ACUGAGAGGCGCAGUCACCCGGUGUGAAGAUGGAC AGCUUUUUAUAUCAUCUUACAAGAAUGAGUAUCAGA CUAUGGAGGUUCAAAAUAACUCCGUGGUUAUAAAG UGCGACGGGCUUUACAUCAUCUAUCUGAAGGGGUC CUUUUUCCAGGAGGUCAAGAUAGAUUUGCACUUCA GGGAAGACCAUAACCCCAUUUCUAUUCCUAUGCUU AAUGACGGACGACGGAUCGUAUUUACUGUUGUGGC UUCUCUCGCUUUUAAGGACAAGGUCUACCUUACCG UGAAUGCCCCAGACACACUUUGCGAGCACCUCCAG AUCAACGACGGAGAGCUUAUCGUUGUUCAACUUAC ACCAGGUUAUUGCGCUCCAGAGGGGAGCUAUCACU CCACCGUCAACCAGGUACCCCUU |
| | 24 | G4S Linker*2 | GGUGGAGGCGGUUCAGGCGGAGGUGGCUCU |
| | 25 | Alpha Helix of MBL | AGCGAGAGAAAGGCCCUUCAGACAGAAAUGGCACG CAUAAAAAAAUGGCUUACAUUCAGU |
| | 24 | G4s Linker | GGUGGAGGCGGUUCA |
| | 26 | Ferritin | CAGGACUCCGAAGCAGCAAUAAACCGACAAAUAAAU CUGGAGUUGUACGCAUCCUACGUUUACCUGUCCAU GUCUUACUACUUCGAUAGAGACGAUGUCGCCCUCA AAAACUUCGCCAAGUAUUUCCUGCAUCAGUCCCAU GAAGAGAGAGAGCACGCAGAGAAACUGAUGAAACU UCAGAACCAACGGGGGGGCAGGAUCUUUCUGCAAG ACAUUAAGAAGCCUGAUUGCGAUGACUGGGAAUCU GGACUUAAUGCCAUGGAGUGUGCCCUGCACCUCGAA AAAAAUGUGAACCAGAGCCUGUUGGAAUUGCAUA AGCUGGCAACCGAUAAGAACGAUCCACAUCUUUGC GAUUUCAUCGAAACCCACUAUCUCAAUGAACAAGUG AAGGCAAUUAAGGAGCUGGGCGAUCAUGUAACAAA UUUGCGAAAAAUGGGUGCC |
| | 13 | 3'UTR | GUGUGUGGAGGACACCCUGAACCCCCCGCUUUCAA ACAAGUUUUCAAAUUGUUUGAGGUCAGGAUUUCUC AAACUGAUUCCUUUCUUUGCAUAUGAGUAUUUGAA AAUAAAUAUUUUCCCAGAAUAUAAAUAAAUCAUCAC AUGAUUAUUUUAACUAU |
| | 14 | Poly(A) Tail | AAAAAAAAAAAAAAAAAAAAAAAACAACCAGCUAAGACAC UGCCAAAAAAAAAAAAAAAAAAAAAAAAAAAAAAAAAAA AAAAAAAAAAAAAAAAAAAAAAAAAAAAAAAAAAAAAAAA AAAAAAAAAAAAAAAAAAAAAAAAAAAAAAAA |
| 4 | 8 | 5'UTR | AGUGCUCCCCAUCCAACUAAACUGUCCCUCUGUCC GAACUAAACUGCACUGUCCCAGCACC |
| | 22 | Signal Sequence | AUGACAAGGUUGACGGUCCUGGCAUUGCUUGCCG GGCUUUUGGCGAGUUCUCGGGCG |
| | 28 | MBL | UCUGGCUCUCAGACUUGUGAAGAUACCCUUAAGAC UUGCAGUGUCAUAGCCUGUGGGCGAGACGGGCGC GAUGGACCCAAGGGCGAAAAAGGAGAACCAGGGCA AGGGCUCAGAGGACUGCAAGGUCCACCUGGUAAAU UGGGGCCACCUGGAAGCGUGGGGUCCCCAGGAAG UCCAGGCCCAAAAGGUCAGAAAGGGGACCACGGCG AUAAUCGAGCCAUUGAGGAAAAACUCGCAAAUAUG GAGGCCGAAAUUAGGAUUCUGAAGAGUAAGUUGCA GCUGACCAACAAGCUCCAUGCUUUUAGCAUGGGC |
| | 24 | G4S Linker*2 | GGUGGAGGCGGUUCAGGCGGAGGUGGCUCU |
| | 23 | Extracellular Domain of OX40L | AGCAGUUCACCUGCAAAGGACCCCCCAAUCCAGCG ACUGAGAGGCGCAGUCACCCGGUGUGAAGAUGGAC AGCUUUUUAUAUCAUCUUACAAGAAUGAGUAUCAGA CUAUGGAGGUUCAAAAUAACUCCGUGGUUAUAAAG UGCGACGGGCUUUACAUCAUCUAUCUGAAGGGGUC CUUUUUCCAGGAGGUCAAGAUAGAUUUGCACUUCA GGGAAGACCAUAACCCCAUUUCUAUUCCUAUGCUU AAUGACGGACGACGGAUCGUAUUUACUGUUGUGGC UUCUCUCGCUUUUAAGGACAAGGUCUACCUUACCG UGAAUGCCCCAGACACACUUUGCGAGCACCUCCAG AUCAACGACGGAGAGCUUAUCGUUGUUCAACUUAC ACCAGGUUAUUGCGCUCCAGAGGGGAGCUAUCACU CCACCGUCAACCAGGUACCCCUU |
| | 13 | 3'UTR | GUGUGUGGAGGACACCCUGAACCCCCGCUUUCAA ACAAGUUUCAAAUUGUUUGAGGUCAGGAUUUCUC AAACUGAUUCCUUUCUUUGCAUAUGAGUAUUUGAA AAUAAAUAUUUCCCAGAAUAUAAAUAAAUCAUCAC AUGAUUAUUUAACUAU |
| | 14 | Poly(A) Tail | AAAAAAAAAAAAAAAAAAAAAAAAACAACCAGCUAAGACAC UGCCAAAAAAAAAAAAAAAAAAAAAAAAAAAAAAAAAAAAA AAAAAAAAAAAAAAAAAAAAAAAAAAAAAAAAAAAAAAAAA AAAAAAAAAAAAAAAAAAAAAAAAAAAAAAAAA |

In Table 8, the extracellular domain of OX40L is, for example, an OX40 receptor-binding domain. In Table 8, G4S linker*2 represents G4SG4S. In the 3' UTR region of SEQ ID NO: 27 described in Table 8, the underlined nucleotides are the Xho I restriction enzyme recognition sequence. In the 3' UTR region of SEQ ID NO: 27 described in Table 8, the bold nucleotides are the Nhe I restriction enzyme recognition sequence. In the poly(A) tail region of SEQ ID NO: 11 described in Table 8, the underlined nucleotides are the Sap I restriction enzyme recognition sequence.

In the poly(A) tail region of SEQ ID NO: 11 described in Table 8, the bold nucleotides are a part of the Eco RI restriction enzyme recognition sequence.

The sequences of Materials 3 and 4 described in Table 8 represent sequences excluding the restriction enzyme recognition sites shown in Materials 1 and 2.

OX40 ligand (OX40L) and OX40 are conventional members of the tumor necrosis factor (TNF) ligand family and the tumor necrosis factor receptor (TNFR) family, respectively. OX40L and OX40 are involved in the costimulation and differentiation of T cells. Like other TNF ligands, OX40L is a type II transmembrane protein. Soluble human OX40L assembles to form a trimer and binds to OX40, but is inactive if not assembled. When Materials 1 and 3 are transcribed and translated, they form a fusion protein having a structure of [Extracellular domain of OX40L]-[G4S linker*2]-[Alpha helix of MBL]-[G4S linker]-[Ferritin]. That is, it is a fusion protein in which the extracellular domain of soluble OX40L is linked to the alpha helix of MBL and ferritin, respectively, through linkers. The fusion protein constitutes a trimeric unit in which three fusion proteins are assembled, and these units may bind to each other to form an oligomer, for example, an oligomer composed of eight units. These oligomers have the activity of binding to and activating OX40. This may facilitate the interaction between the extracellular domain of OX40L and OX40 more effectively than when the soluble OX40L is expressed alone. In the fusion protein, the alpha helix of MBL may promote the formation of the trimeric units of the fusion protein. In addition, in the fusion protein, ferritin may promote the formation of oligomers by binding the formed trimeric units of the fusion protein to each other. The oligomer may have a cage-like form. In the fusion protein, the extracellular domain of OX40L may be any polypeptide including the extracellular domain of OX40L, for example, full-length OX40L. However, the claimed invention should not be construed as being limited to such a specific mechanism.

When Materials 2 and 4 are transcribed and translated, they form a fusion protein having a structure of [MBL]-[G4S linker*2]-[Extracellular domain of OX40L]. That is, it is a fusion protein in which the extracellular domain of soluble OX40L is linked to MBL through a linker. The fusion protein constitutes a trimeric unit in which three fusion proteins are assembled, and these units may bind to each other to form an oligomer, for example, an oligomer composed of 1, or 2 to 4 or more units of the trimer. These oligomers can bind to and activate OX40. This may facilitate the interaction between the extracellular domain of OX40L and OX40 more effectively than when the soluble OX40L is expressed alone. In the fusion protein, MBL may promote the formation of the trimeric units of the fusion protein. In addition, in the fusion protein, MBL may promote the formation of oligomers by binding the formed trimeric units of the fusion protein to each other. The oligomer may have a bouquet-like structure. In the fusion protein, the extracellular domain of OX40L may be any polypeptide including the extracellular domain of OX40L, for example, full-length OX40L. However, the claimed invention should not be construed as being limited to such a specific mechanism.

**[Table 9]**

| SEQ ID NO: | Amino Acid Sequence |
|---|---|
| 29 | MTRLTVLALLAGLLASSRASSSPAKDPPIQRLRGAVTRCEDGQLFISSY KNEYQTMEVQNNSVVIKCDGLYIIYLKGSFFQEVKIDLHFREDHNPISIPM LNDGRRIVFTVVASLAFKDKVYLTVNAPDTLCEHLQINDGELIVVQLTPG YCAPEGSYHSTVNQVPLGGGGSGGGGSSERKALQTEMARIKKWLTFS GGGGSQDSEAAINRQINLELYASYVYLSMSYYFDRDDVALKNFAKYFLH QSHEEREHAEKLMKLQNQRGGRIFLQDIKKPDCDDWESGLNAMECAL HLEKNVNQSLLELHKLATDKNDPHLCDFIETHYLNEQVKAIKELGDHVTN LRKMGA |
| 30 | MTRLTVLALLAGLLASSRASGSQTCEDTLKTCSVIACGRDGRDGPKGE KGEPGQGLRGLQGPPGKLGPPGSVGSPGSPGPKGQKGDHGDNRAIE EKLANMEAEIRILKSKLQLTNKLHAFSMGGGGSGGGGSSSSPAKDPPI QRLRGAVTRCEDGQLFISSYKNEYQTMEVQNNSVVIKCDGLYIIYLKGS FFQEVKIDLHFREDHNPISIPMLNDGRRIVFTVVASLAFKDKVYLTVNAP DTLCEHLQINDGELIVVQLTPGYCAPEGSYHSTVNQVPL |

In Table 9, SEQ ID NOs: 29 and 30 are amino acid sequences obtained by the translation of the sequences of Materials 3 and 4, respectively.

## Claims

1. An mRNA molecule comprising a modified polyadenylation sequence, wherein the modified polyadenylation sequence comprises:
(a) a first sequence comprising one or more microRNA binding sequences;
(b) a second sequence of 20 or fewer consecutive A nucleotides, located at a 5' end of the first sequence; and
(c) a third sequence of 80 or more consecutive A nucleotides, located at a 3' end of the first sequence.

2. The mRNA molecule of claim 1, wherein the microRNA binding sequence of the first sequence is tumor-specific.

3. The mRNA molecule of claim 2, wherein the microRNA is selected from the group consisting of miRNA-34a, miR-340, miR-133a, miR-34b, miR-19, miR-200, miR-15, miR-16, miR-1298, miR-663a, miR-449a, miR-138, miR-126, miR-10a, miR-10b, miR-140-3p, miR-140-5p, miR-204, miR-424, miR-193a-3p, miR-455-5p, miR-455-3p, miR-9, miR-10a, miR-148a, miR-488, miR-196a1, miR-182, miR-96, miR-193b, miR-27a, miR-196b, miR-10b, miR-29b2, miR-23a, miR-107, miR-542-3p, miR-93, miR-365a-4, miR-450a, miR-100, miR-105, miR-363, miR-105, miR-106b, miR-15b, miR-21, miR-376c, miR-93, miR-99b, miR-155, miR-33a, miR-876-3p, miR-362-3p, miR-25, let-7i, miR-423-3p, miR-16-2, miR-29a, miR-30d, miR-320, miR-181c, miR-128a, miR-21, let-7d, miR-450b-5p, miR-371-5p, miR-1245, miR-335, miR-492, miR-874, miR-30b, miR-193a-5p, miR-602, miR-346, miR-663, miR-25, miR-219-5p6, miR-184, miR-135a7, miR-584, miR-665, miR-638, miR-503, miR-628-3p, miR-381, miR-78, miR-92b, miR-149, miR-135b, miR-302d, miR-498, miR-766, miR-1389, miR-623, miR-519c-5p, miR-182, miR-494, miR-129-5p10, miR-513-5p, miR-200b, miR-634, miR-654-5p, miR-518b, miR-658, miR-373, miR-30c-2, miR-130a, miR-557, miR-551a, miR-637, miR-518c, miR-525-5p, miR-596, miR-552, miR-625, miR-183, miR-187, miR-544, miR-891a, miR-519e, miR-933, miR-939, miR-214, miR-671-5p, miR-137, miR-92b, miR-525-3p, miR-19a, and miR-409-5p.

4. The mRNA molecule of claim 1, comprising one or more modified nucleotides.

5. The mRNA molecule of claim 1, further comprising one or more sequences selected from the group consisting of a sequence encoding a polypeptide and an expression regulatory sequence.

6. The mRNA molecule of claim 5, wherein the expression regulatory sequence comprises one or more selected from the group consisting of a Kozak sequence, a 5' UTR, and a 3' UTR.

7. The mRNA molecule of claim 6, wherein the 5' UTR is derived from an mRNA encoding a protein selected from the group consisting of p53, OX40L, albumin, serum amyloid A, apolipoprotein A/B/E, transferrin, alpha-fetoprotein, erythropoietin, Factor VIII, MyoD, myosin, myoglobin, myogenin, herculin, Tie-1, CD36, C/EBP, AML1, G-CSF, GM-CSF, CD11b, MSR, Fr-1, i-NOS, CD45, CD18, CD36, GLUT4, ACRP30, adiponectin, P-A/B/C/D, ORM1, HPX, FGA, CYP2E1, C3, ASC, APOA2, ALB, AGXT, α-globin, β-globin, tyrosine hydroxylase, and collagen.

8. The mRNA molecule of claim 6, wherein the 3' UTR is derived from an mRNA encoding a protein selected from the group consisting of: p53, OX40L, albumin, serum amyloid A, apolipoprotein A/B/E, transferrin, alpha-fetoprotein, erythropoietin, Factor VIII, MyoD, myosin, myoglobin, myogenin, herculin, Tie-1, CD36, C/EBP, AML1, G-CSF, GM-CSF, CD11b, MSR, Fr-1, i-NOS, CD45, CD18, CD36, GLUT4, ACRP30, adiponectin, P-A/B/C/D, ORM1, HPX, FGA, CYP2E1, C3, ASC, APOA2, ALB, AGXT, α-globin, β-globin, tyrosine hydroxylase, and collagen.

9. The mRNA molecule of claim 1, wherein the modified polyadenylation sequence comprises 90 to 1125 nucleotides.

10. A composition for delivering mRNA or a product expressed therefrom to a subject, comprising the mRNA of any one of claims 1 to 9 as an active ingredient.

11. A method of delivering mRNA or a product expressed therefrom to a host cell, comprising introducing the mRNA of any one of claims 1 to 9 into the host cell.

12. A DNA molecule encoding the mRNA molecule of any one of claims 1 to 9.

13. The DNA molecule of claim 12, further comprising an expression regulatory sequence operably linked to the DNA molecule, wherein the expression regulatory sequence comprises a T7, T3, or SP6 promoter.

14. The DNA molecule of claim 12, wherein the DNA molecule is a nucleic acid construct or a vector.
